# EUROPEAN PATENT SPECIFICATION

(11) **EP 3 368 556 B1**
(45) Date of publication and mention of the grant of the patent: **10.04.2024**
(21) Application number: 16860981.6
(22) Date of filing: 28.10.2016
(51) Int. Cl.: C07K 14/605, A61K 38/00, A61K 38/26, A61P 3/10

(54) **NOVEL POLYPEPTIDES WITH IMPROVED PROTEOLYTIC STABILITY, AND METHODS OF PREPARING AND USING SAME**
NEUARTIGE POLYPEPTIDE MIT VERBESSERTER PROTEOLYTISCHER STABILITÄT UND VERFAHREN ZUR HERSTELLUNG UND VERWENDUNG DAVON
NOUVEAUX POLYPEPTIDES PRÉSENTANT UNE MEILLEURE STABILITÉ ET PROCÉDÉS DE PRÉPARATION ET D'UTILISATION ASSOCIÉS

(30) Priority: 28.10.2015 US 201562247493 P
(43) Date of publication of application: 05.09.2018
(73) Proprietor: Tufts University, Medford, MA 02155 (US); Tufts Medical Center, Inc., Boston, MA 02111 (US)
(72) Inventor: KUMAR, Krishna, Medford, Massachusetts 02155 (US); MONTANARI, Vittorio, Medford, Massachusetts 02155 (US); BEINBORN, Martin, Boston, Massachusetts 02111 (US); RAMAN, Venkata, Medford, Massachusetts 02155 (US)
(74) Representative: Fleuchaus & Gallo Partnerschaft mbB
(86) International application number: PCT/US2016/059541
(87) International publication number: WO 2017/075505

(56) References cited:
- WO-A2-2008/128064
- WO-A2-2015/086686
- US-A1- 2011 166 321
- US-A1- 2015 045 281
- H LIU: "N-acetyl-GLP-1: a DPP IV-resistant analogue of glucagon-like peptide-1 (GLP-1) with improved effects on pancreatic &bgr;-cell-associated gene expression", CELL BIOLOGY INTERNATIONAL, vol. 28, no. 1, 1 January 2004 (2004-01-01), pages 69-73, XP055181637, ISSN: 1065-6995, DOI: 10.1016/j.cellbi.2003.10.004
- VISHAL GUPTA: "Glucagon-like peptide-1 analogues: An overview", INDIAN JOURNAL OF ENDOCRINOLOGY AND METABOLISM, vol. 17, no. 3, 1 January 2013 (2013-01-01), page 413, XP055154779, ISSN: 2230-8210, DOI: 10.4103/2230-8210.111625
- GALLWITZ et al.: "GLP-1-analogues resistant to degradation by dipeptidyl-peptidase IV in vitro", Regul Pept., vol. 86, no. 1-3, 2000, pages 103-11, XP002292372,
- ORSKOV: "Glucagon-like peptide-1, a new hormone of the entero-insular axis", Diabetologia, vol. 35, no. 8, 1992, pages 701-11, XP055541323,
- ARAI et al.: "The Conformation of de Novo Designed Amphiphilic Peptides with Six or Nine L-2- (2,2,2-Trifluoroethyl)glycines as the Hydrophobic Amino Acid", Bulletin of the Chemical Society of Japan, vol. 73, no. 2, 2000, pages 439-445, XP002320762, DOI: doi:10.1246/bcsj.73.439

## Description

### BACKGROUND OF THE INVENTION

Polypeptides have attracted much interest as therapeutic agents. They have proven biological activity, have very high affinity and exquisite specificity for their biological targets, and can be prepared in large scale using recombinant techniques or chemical synthesis. However, polypeptide-based therapeutics face key liabilities, such as poor *in vivo* stability and short *in vivo* half-life. Polypeptides are substrates for several *in vivo* peptidases, such as dipeptidyl protease 4 (DPP4) and endopeptidases, which cleave the polypeptide chain into fragments that usually exhibit diminished function. Further, polypeptides may be chemically modified within the body, marking them for excretion and/or degradation.

Attempts to minimize the instability of polypeptides towards proteolysis have been met with limited success. Acetylation of the N-terminus amino group of the polypeptide has the general effect of reducing proteolysis rates, but with accentuated loss in biological activity. Further, the N-acetyl group is still far from being stable, undergoing hydrolysis *in vivo.* Stabilization against enzymes such as DPP4 may be achieved by replacing amino acids at the target cleavage site, but this modification also leads to concomitant loss of biological activity in most cases.

Drawbacks of peptides as developable therapeutics, especially short plasma half-life times, have been addressed by targeted chemical modifications. For instance, insertion of unnatural amino acids in the peptide chain is a common first-pass approach to new peptide lead generation. However, modifying a biologically active polypeptide at certain main chain positions can result in unacceptable loss of activity. This process of structural modification is also largely a matter of trial and error. Combined with the intrinsic low yields of chemical synthesis and peptide isolation, target chemical modification is a very expensive approach.

GLP-1 is a potent 3 1-residue peptide secreted by enteroendocrine cells in response to food ingestion, and possesses the salient properties of delayed gastric emptying and induced satiety, insulin secretion upon glucose intake, increase of β-cell mass and function, and concomitant weight loss. However, its half-life *in vivo* is less than two minutes, due to the action of endogenous serine protease dipeptidyl protease 4 (DPP4), making GLP-1 unsuitable for the treatment of Type II diabetes. Only two GLP-1 analogs have emerged as important therapeutics: Exenatide, a structural GLP-1 analog; and Liraglutide, which is a derivative of GLP-1 with an added large lipid side chain. Both of these drugs were developed in an effort to improve the proteolytic stability of GLP-1.

Liraglutide self assembles into heptamers in solution, and shows enhanced albumin binding, partially protecting it from hydrolytic cleavage by DPP4. However, despite its unsurpassed ability among FDA-approved GLP-1 mimetics to reduce blood glucose in diabetics and induce weight loss, liraglutide is a relatively short acting drug that needs daily injections. Further, liraglutide is still markedly degraded by DPP4 leading to its inactivation.

The derivative N-acetyl-GLP-1 is more stable to DPP4 degradation, but is 10-50 fold less active than GLP-1 itself. Thus, N-acetyl-GLP-1 cannot be used as a therapeutic agent. Moreover, N-acetylation confers *in vitro* stability against DPP4, but may not be effective against exopeptidases that are ubiquitous in serum. Further, deacetylation catalyzed by dedicated enzymes may occur *in vivo.*

Additional approaches to further extend the half-life of GLP-1 have relied on fusing the polypeptide to large carrier proteins such as albumin (albiglutide) or immunoglobulin (dulaglutide). However, probably due to reduced CNS access, these constructs are less effective than liraglutide in reducing weight. Alternatively, the penultimate residue (Ala8) in GLP-1 was replaced with a helix-inducing non-canonical amino acid (α-aminoisobutyric acid, Aib). A corresponding derivative (taspoglutide) triggered antibody formation like exenatide in addition to injection site reactions, and was therefore withdrawn during clinical trials.

Liu, H., (Cell Biology International, vol. 28, no. 1, 2004, pages 69-73) describes the ability of a glucagon-like peptide (GLP-1) to regulate the blood glucose and a therapeutic potential in diabetes.

Gallwitz B. et al., (Regul Pept., vol. 86, no. 1-3, 2000, pages 103-11) describes that a GLP-1 is used as a therapeutic option for type 2 diabetics with residual β-cell function that stimulates insulin secretion and inhibits glucagon secretion. It is expected to have an extended biological action and a higher potency in vivo but that fact is not proven and further experiments have to be done thus the cited document struggles with short biological half lives.

In the patent application US 2015/045281 A1 GLP-1 prodrugs are described for the treatment and/or prevention of forms of diabetes and related diseases by improving B-cell function and/or delaying or preventing diabetic disease progression. The duration of action of GLP-1 is claimed to be extended to improve the half-lives of the modified compounds. In the patent application WO 2008/128064 A2 the text relates to biologically active truncated peptides and P'₁ analogs with claimed. Chemical modifications with non-naturally occurring amino acids and steric and electronic nature like Secretin, GLP-1, GLP-2, GLP-1(7-36) amide which should reduce the blood glucose levels and improve the glucose metabolism in mammals are described.The patent application WO 2015/086686 A2 describes methods for making protease resistant peptides and synthetic (incretin class) peptides like alpha-methyl functionalized amino acids and substituents (like alpha-methyl phenylalanine) bound to the alpha carbon atom and a method for utilizing such peptides.

However, all these approaches are struggling with short or still too short biological half lives that limit its use in therapeutic approaches.

There is a pressing need to develop more efficacious treatments for patients with short bowel syndrome (SBS), a debilitating medical condition resulting from an inability to adequately absorb nutrients from the intestine. This orphan disease occurs in patients who undergo a major loss of small bowel with surgery. A variety of underlying pathologies may lead to SBS including Crohn's disease, trauma, congenital abnormalities, and malignancy. Short bowel syndrome results in severe diarrhea, dehydration and malnutrition requiring parenteral nutrition (PN) for survival, delivered via an indwelling venous catheter. Complications of PN include sepsis, venous thrombosis and metabolic liver disease. The cumulative cost of SBS associated PN in the USA including hospitalizations is on the order of $5 billion per year.

Post-surgery, there is a prolonged period, up to 2 years, where the remnant intestine adapts and nutrient absorption increases. The physiological compensation after adaptation, however, is often inadequate to meet even minimal nutritional requirements. Research efforts have focused on the identification of pharmacological agents that enhance this adaptive process. Administration of Glucagon-like Peptide-2 (GLP-2) acting through its cognate GPCR (GLP-2R) triggers expansion of the intestinal epithelium, resulting in increased nutrient absorption, enhanced barrier function and increased blood flow.

A modified GLP-2 peptide, teduglutide or "GATTEX^{®}", was FDA approved in 2012 for the treatment of SBS. GATTEX^{®} includes a single amino acid alteration in position 2 (His-Gly-, instead of His-Ala), which enhances resistance of the polypeptide to dipeptidyl peptidase-4 (DPP4) extending its *in vivo* half-life from 7 minutes to ~3 hours. GATTEX^{®} was an important but limited therapeutic step forward. After 52 weeks of treatment, 68% of SBS patients were able to reduce their PN regimen by at least 1 day/week. Despite this progress, only 4 of 52 treated patients became entirely independent of PN, and 32% failed to reduce their PN regimen by even 1 day/week. Yet, the cost of GATTEX^{®} per patient is ~$300,000/year. Further, introduction of the Gly residue at position 2 leads to formation of an unexpected synthetic impurity (aspartimide contamination), which must be avoided by using a manufacturing workaround that renders the synthesis longer and more expensive. Thus, more effective/competing drugs, including improved GLP-2 analogs, are critically needed for SBS to improve patient quality of life and to reduce treatment costs.

There is a need in the art to identify novel methods of improving proteolytic stability of polypeptides without negatively affecting their biological activities. The present invention meets this need.

### SUMMARY OF THE INVENTION

The present invention relates to chemically modified polypeptides, salts or solvates thereof which are characterized with an increased proteolytic stability without negative effects on their biological activities. In some embodiments, the chemically modified polypeptides, or salts or solvate thereof, wherein the polypeptide may have the chemical modifications as of claim 1, namely:
(i) at least one selected from the chemically modified GLP-1 polypeptide comprising an amino acid sequence having at least 75% identity to GLP-1 (H*AEGTFTS*DVS*S*YLEGQAAK*EFIAWLVK*GR), wherein at least one of the amino acid residues marked with an asterism * is alkylated with an optionally substituted C₁-C₁₆ alkyl group, wherein the N-terminal amino acid residue of the polypeptide is an N-2,2,2-trifluoroethyl amino acid represented by the formula: 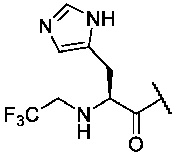 or 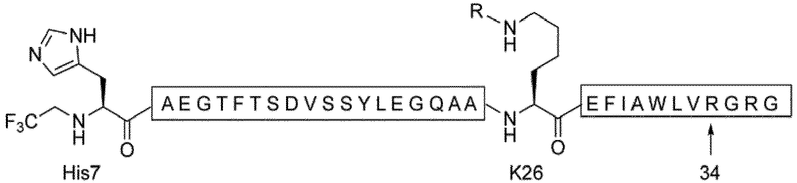 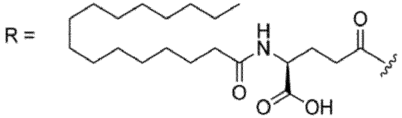
(ii) The chemically modified polypeptides may have greater serum stability as compared to a corresponding non-chemically modified polypeptide. The chemically modified polypeptide may have longer *in vivo* half-life as compared to the corresponding non-chemically modified polypeptide. The chemically modified polypeptide of claim 1 may have greater blood-brain barrier permeability or greater oral bioavailability as compared to the corresponding non-chemically modified polypeptide. In some embodiments, the modified polypeptide's half-life may be increased by at least 10-fold relative to the corresponding non-chemically modified polypeptide. In some embodiments, the modified peptide may retain at least about 5% of the biological activity of the corresponding non-chemically modified polypeptide.

The polypeptide may be further selected from the chemically modified polypeptide comprising an amino acid sequence having at least 75% identity to GLP-1(7-37) (H* AEGTFTS *DVS * S *YLEGQAAK *EFIA WL VK *GRG-OH) wherein the lysine residue at position 26 (K26) has a lipide side chain with γ-Glu-palmitoyl; and lysine 34 (K34) is modified to an arginine residue (R34).

A pharmaceutical composition comprising the chemically modified polypeptide is also provided.

Further disclosed are the polypeptides for use in a method of treating or preventing at least one disease or disorder. the methods may comprise administering at least one chemically modified polypeptide to a patient in need thereof. theThe methods may comprise administering pharmaceutical compositions comprising any chemically modified polypeptide described herein. theThe disease or disorder may be selected from the group consisting of short bowel syndrome, non-alcoholic steatohepatitis, smoking cessation, neurodegeneration, Alzheimer's disease, Parkinson's disease, congenital hyperinsulism, and/or hypoglycemia. In some embodiments, the disease or disorder may be selected from the group of diabetes, weight gain, obesity, and/or metabolic syndrome.

Methods of increasing the *in vivo* half-life of polypeptides and/or improving the blood-brain barrier permeability of polypeptides and/or improving oral bioavailability of a polypeptide are also provided. These changes (*e.g*., increase half-life, improve permeability, improve bioavailability) are compared to a corresponding non-chemically modified polypeptide.
As described, these methods may comprise any chemical modification . As described, the method increasing the *in vivo* half-life of polypeptides and/or improving the blood-brain barrier permeability of polypeptides and/or improving oral bioavailability as compared to the corresponding non-chemically modified polypeptide.

Methods of imaging a cell or tissue in a subject are also possible. In some embodiments, the method of imaging a cell or tissue in a subject comprising administering to the subject in need thereof an effective amount of a (chemically modified) polypeptide, wherein the polypeptide is labeled with a detectable isotope and/or conjugated to a detectable label. Further, the detectable label may comprise a chromophore, a fluorescent group, a bioluminescent group, a chemiluminescent group or a radioactive group.

### BRIEF DESCRIPTION OF THE DRAWINGS

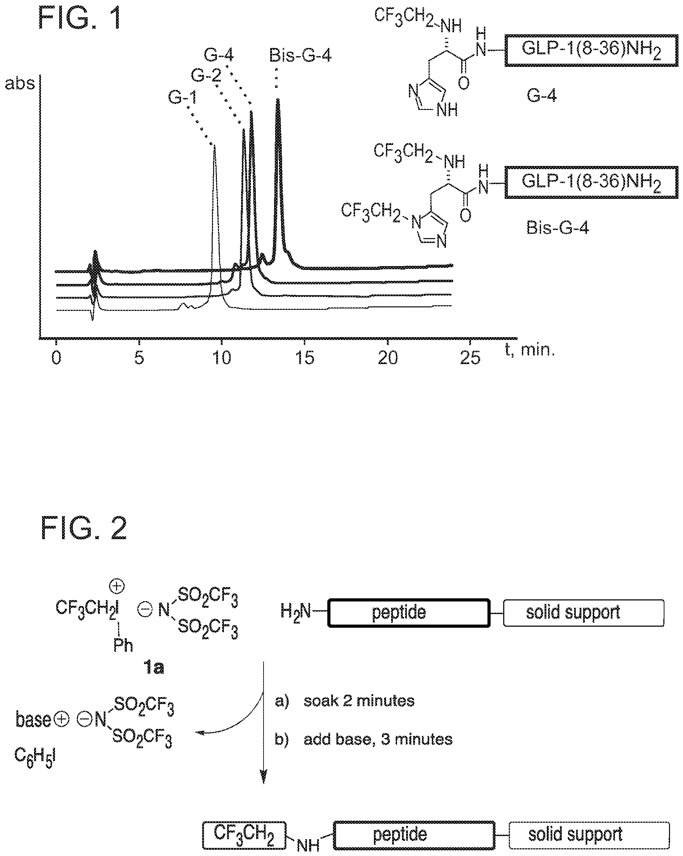

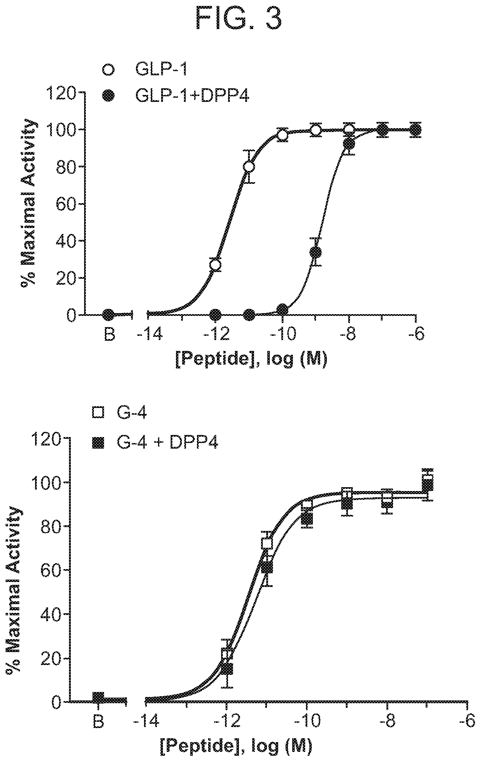

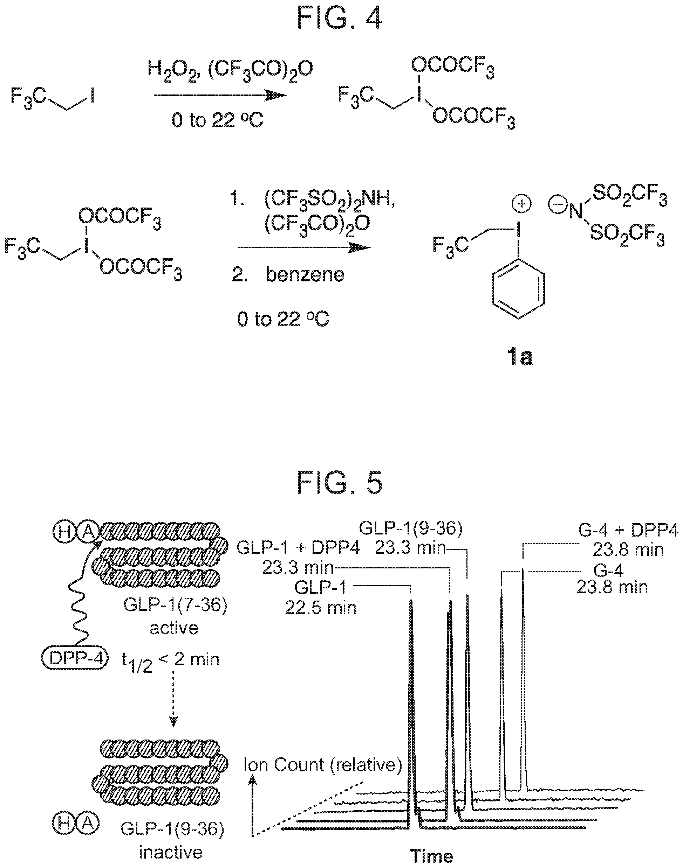

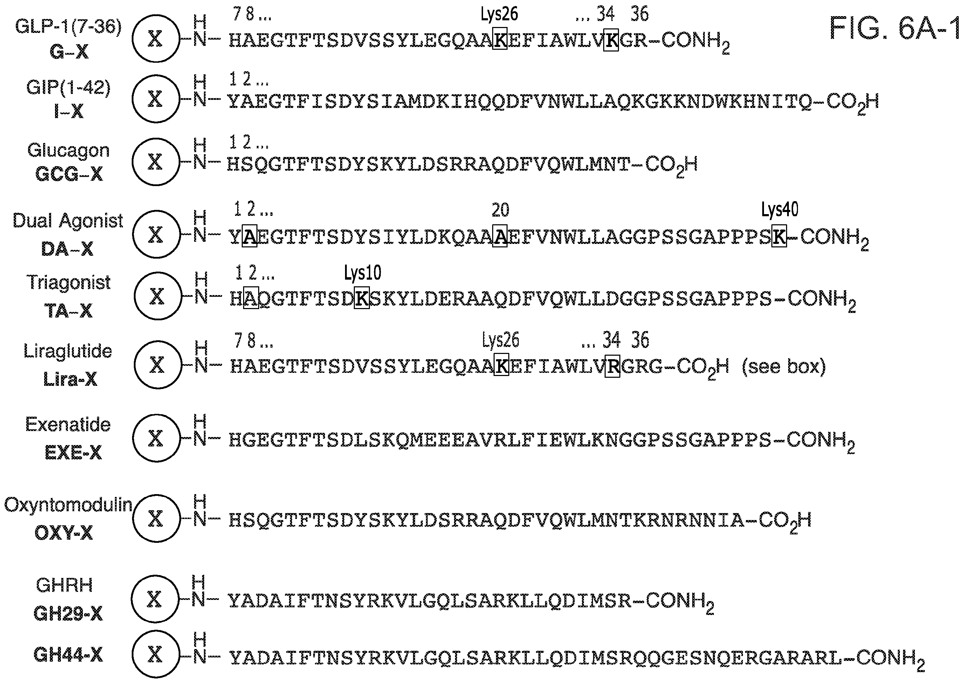

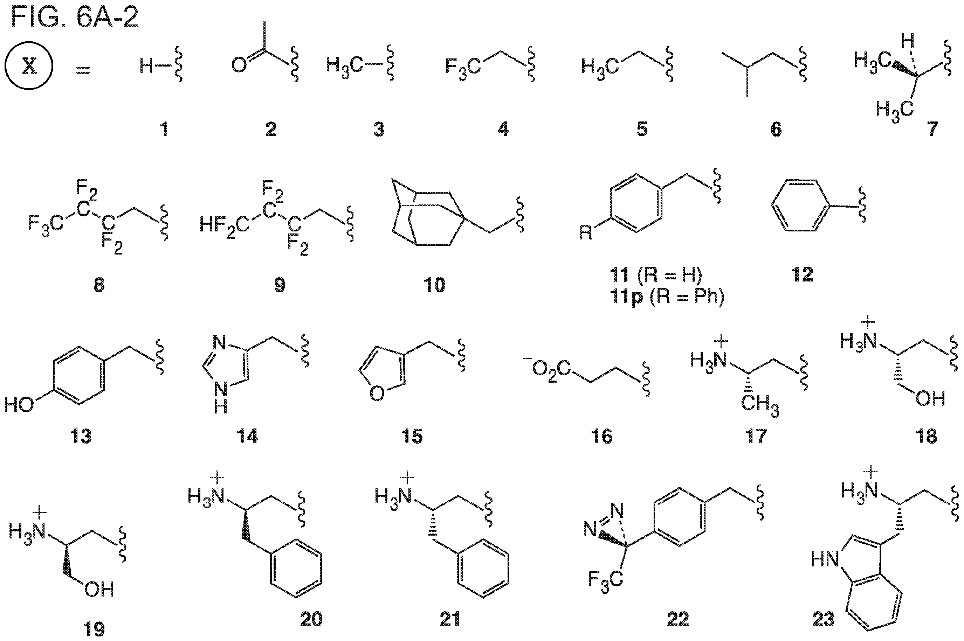

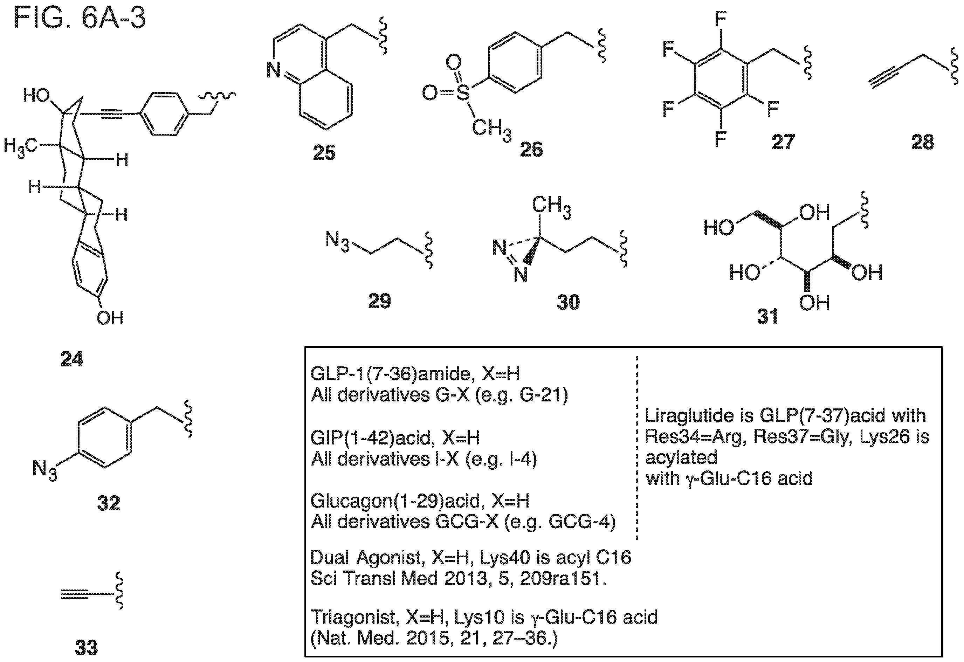

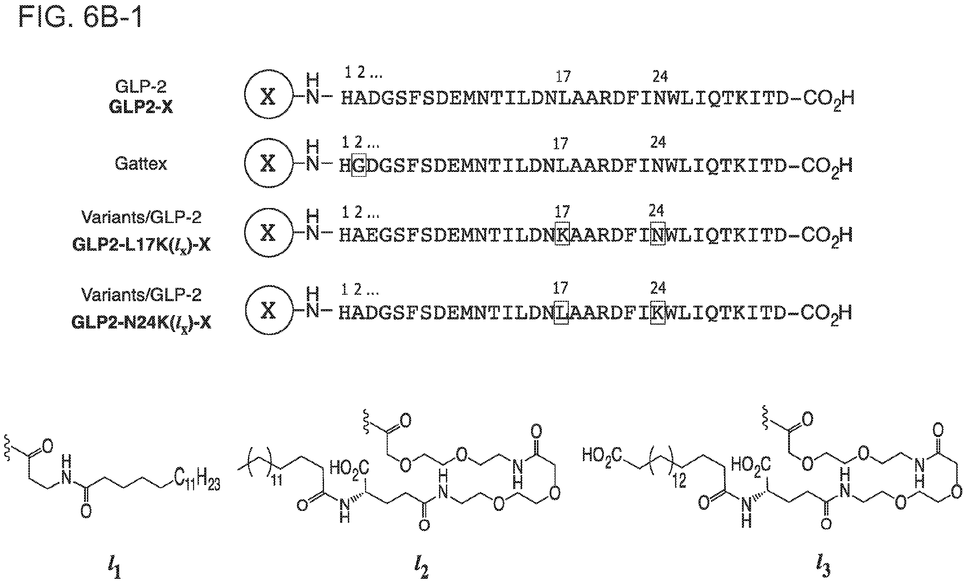

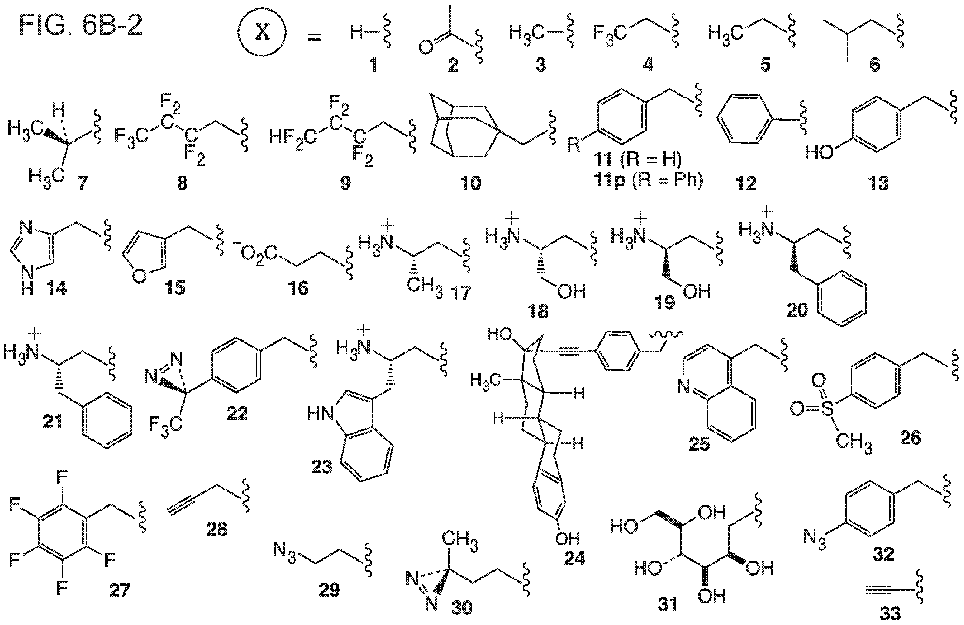

The following detailed description of specific embodiments of the invention will be better understood when read in conjunction with the appended drawings. For the purpose of illustrating the invention, specific embodiments are shown in the drawings
FIG. 1 provides HPLC traces of native and modified GLP-1 variants, which were obtained concurrently from the same batch of resin in SPPS. Two products were observed by direct on-resin alkylation of GLP-1 with reagent **1a** (see Figs. 2 and 4), which correspond to N-terminal decoration of GLP-1 with either one CHCF3 group (G-4) or with two of these moieties ("Bis-G-4"). In the latter case, the second alkylation occurs on the *π* nitrogen of His. Peptides were purified to >95% purity before they were assayed against the GLP-1 receptor (FIG. 3). Nomenclature according to Fig. 6 is as follows: G-1 (unmodified GLP-1; G-2 (Ac-GLP-1); G-4 (mono-C2-GLP-1). "Bis-C2-GLP-1" is GLP-1 with two CH2CF3 moities attached to the N-terminal histidine.
FIG. 2 provides a schematic showing of an exemplary fluoroalkylation scheme. The trivalent iodonium salt coordinates free amines, and the addition of a base triggers the alkylation reaction. The resulting trifluoroethyl function is unreactive, and different chemical modifications of side chains can be carried out in its presence, either on or off-resin. At the end of the synthesis, the peptide is cleaved from the resin and worked up as usual.
FIG. 3 provides data from exemplary stability assays for native and fluoroalkylmodified GLP-1 ("G-4" in nomenclature according to Fig. 6). Briefly, HEK293 cells were plated at a density of 2-3×10³ cells per well onto clear-bottom, white, 96-well plates and grown for 2 days to ~80% confluency. Cells were then transiently transfected by using Lipofectamine reagent (Invitrogen) with cDNA encoding (1) a GPCR (or empty expression vector); (ii) a cAMP-responsive element-luciferase reporter gene (CRE₆ₓ-luc), and (iv) β-galactosidase as a control. Cells were incubated with or without the selected peptide agonist in serum-free medium for 6 h. Luciferase activity was quantified by using Steadylite reagent (Perkin-Elmer). A β-galactosidase assay then was performed after adding the enzyme substrate 2-nitrophenyl β-d-galactopyranoside. Following incubation at 37 °C for 30-60 min, substrate cleavage was quantified by measurement of OD at 420 nM using a SpectraMax microplate reader (Molecular Devices). Top panel: Native GLP-1 is degraded after incubation with DPP4, resulting in a pronounced potency loss compared to GLP-1 without DPP4 incubation. Bottom panel: In contrast, G-4 is completely resistant to DPP4-induced degradation.
FIG. 4 provides a schematic showing the synthesis of a reagent useful within the methods of the invention.
FIG. 5 provides a set of images showing that GLP-1 is rapidly deactivated by DPP4, an enzyme that cleaves the dipeptide HA from the N-terminus. Left: Cartoon depicting the inactive peptide that has lost the amino terminal HA dipeptide fragment. Right: ESI LC-MS analysis of GLP-1 and G-4 (an N-trifluoroethyl analogue that is unyielding to enzyme action) incubated overnight with DPP4 at 37 °C.
FIG. 6A provides a schematic representation of chemical structures of certain analogues of the invention. X corresponds to moieties at the N-terminus, as represented by numbers. All unmodified peptides have X=1 (corresponding to hydrogen; e.g. unmodified GLP-1 = G-1). Abbreviated nomenclature used in the text and in other figures is as follows: G-X (GLP-1 analogues); I-X (GIP analogues); GCG-X (glucagon analogues); DA-X (dual agonist analogues); TA-X (triagonist analogues); Lira-X (liraglutide analogues); EXE-X (exendin analogues); OXY-X (oxyntomodulin analogues); GH29-X or GH44-X (growth hormone releasing hormone analogues).
FIG. 6B provides a schematic representation of chemical structures of GLP-2 analogues of the invention. X corresponds to moieties at the N-terminus, as represented by numbers. Peptides that are unmodified at the N-terminus have X=1 (corresponding to hydrogen; e.g. unmodified GLP-2 = GLP2-1). Some of the analogues are additionally modified by a lysine substitution and attachment of a lipid-linker moiety in this position. Examples for GLP-2 positions where such modifications are made include L17 and N24, and examples of attachments include *l1, l2,* or *l3*. Gattex is an established GLP-2 based drug that includes an A2G substitution.

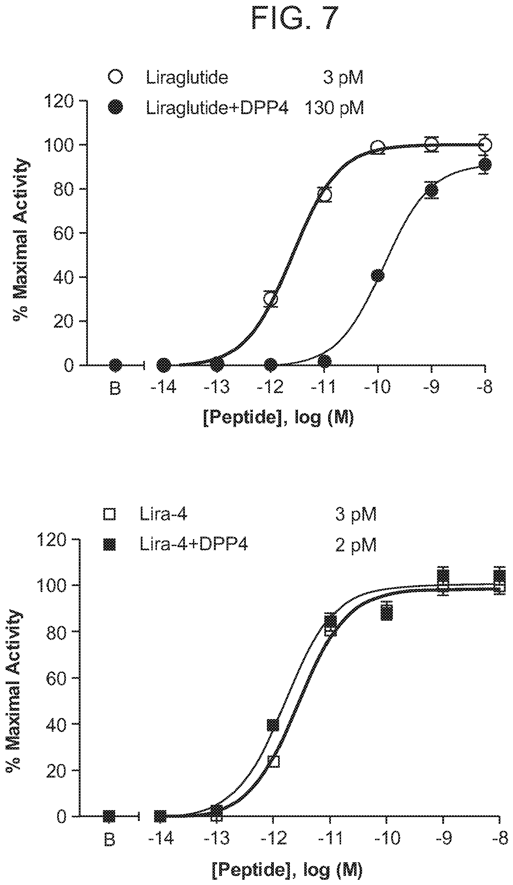

FIG. 7 provides a graph showing concentration response curves of liraglutide and Lira-4 (see FIG. 6, corresponds to N-trifluoroethyl modified liraglutide), with and without DPP4 incubation overnight. Under these conditions, >98% of liraglutide was degraded while Lira-4 remained intact. Corresponding EC50 values are indicated in pM.

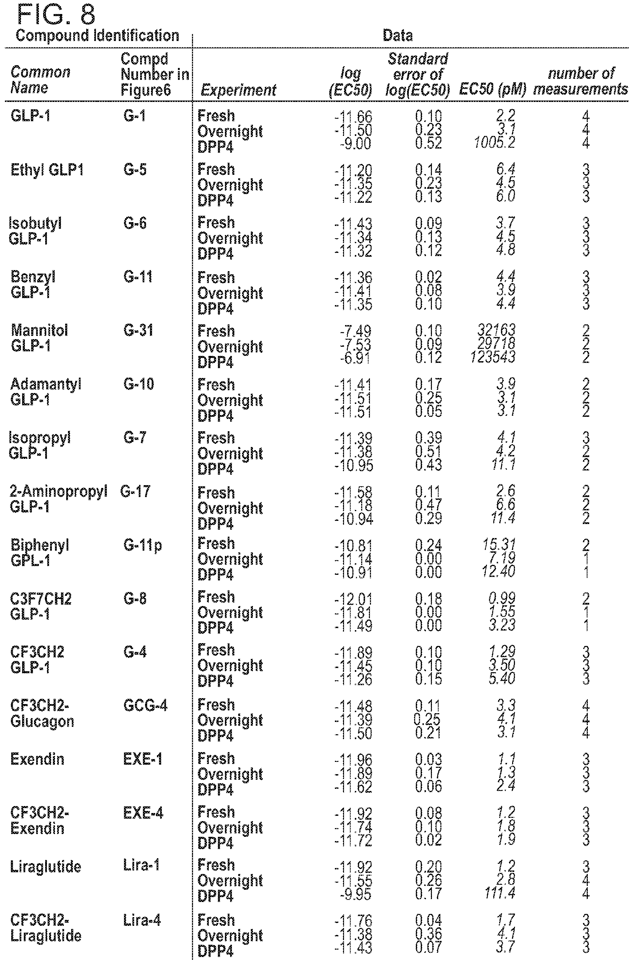

FIG. 8 provides a table summarizing exemplary results of stability assays (incubated with or without DPP4) for certain GLP-1 analogues.

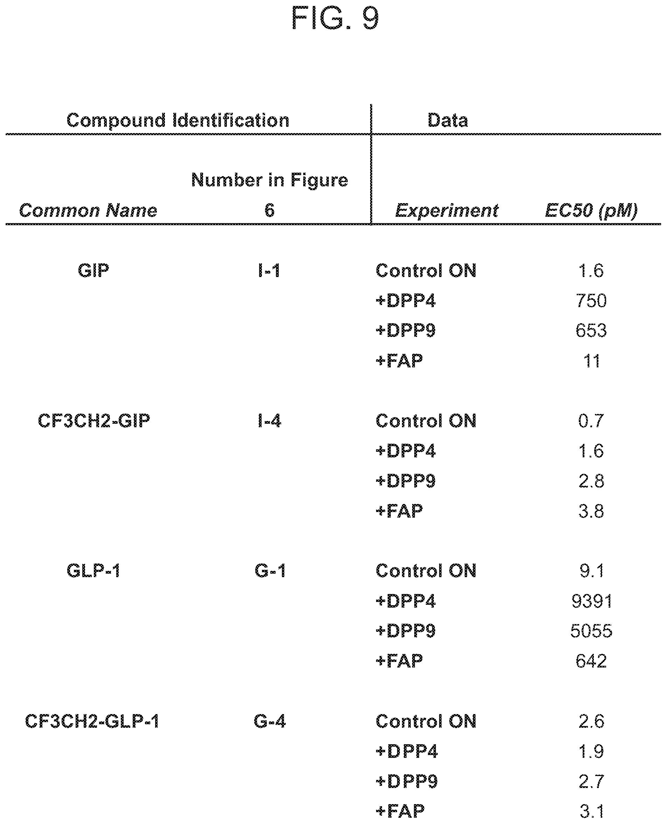

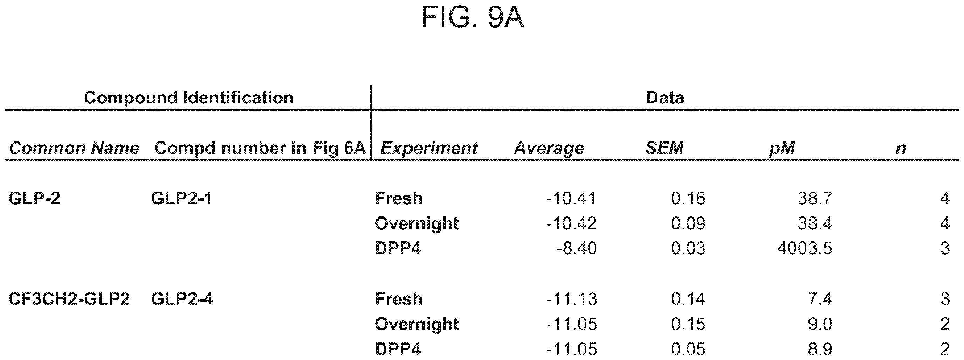

FIG. 9A provides a table summarizing exemplary results of stability assays (incubated with or without DPP4, DPP2, or FAP) for certain GLP-1 or GIP. Whereas native GLP-1 (G-1) and GIP (I-4) show major potency losses due to enzyme exposure (reflected by an increase in EC50), corresponding alkylated analogues (G4 and I4, respectively) are stable under the same conditions.

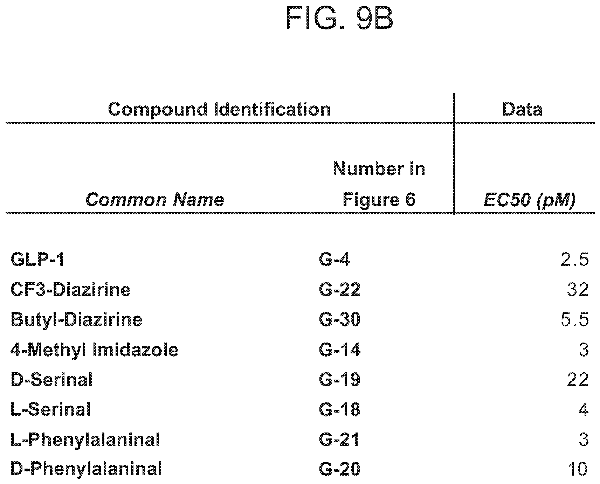

FIG. 9B provides a table showing that native GLP-2 (GLP2-1) is degraded by DPP4 whereas a trifluoroethyl decorated analog (GLP2-4) is completely resistant to degradation. Peptides were incubated with vehicle or recombinant DPP4. Serial dilutions of GLP2-1 and GLP2-4 were then applied to HEK293 cells, which had been transfected with cDNA encoding human GLP-2R and a cAMP-responsive reporter gene. DPP4 induced a major increase in the EC50 of GLP-2 (indicating reduced potency), which was not observed with the GLP2-4 derivative.
FIG. 9C provides a table showing that selected N-terminal decorations are well tolerated in GLP-1. Compound potencies were assessed by bioassay in HEK293 cells expressing GLP-1 receptors and a cAMP-responsive luciferase reporter gene.

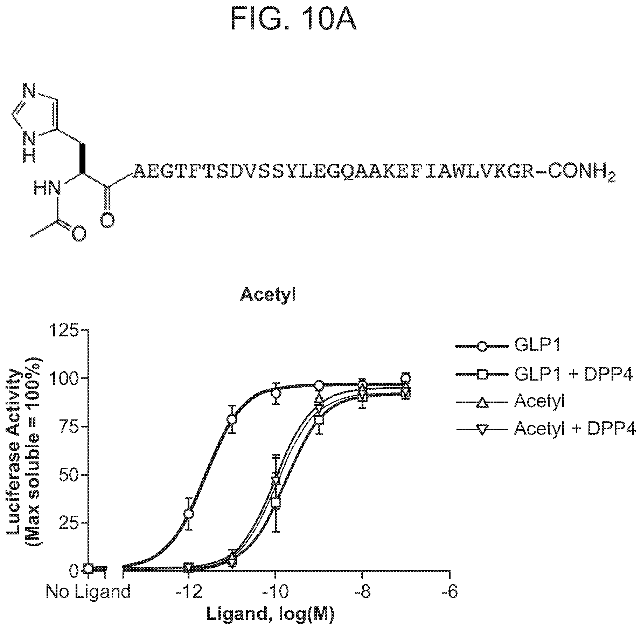

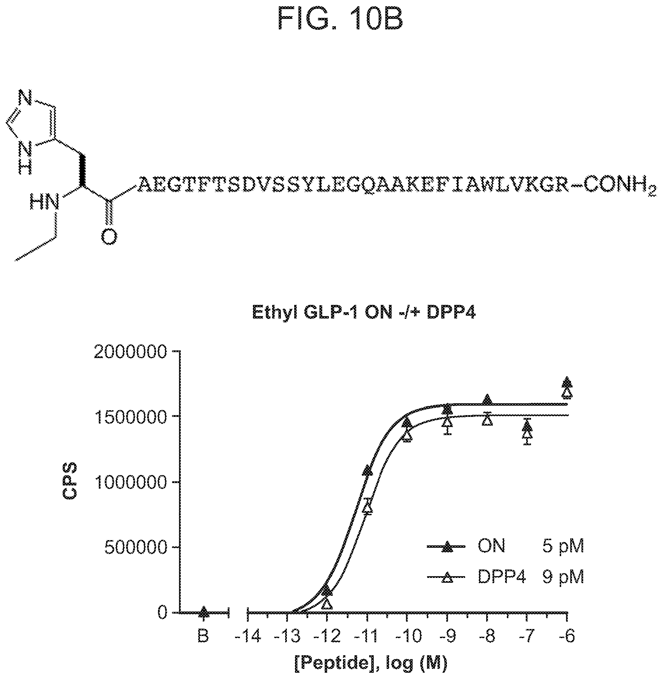

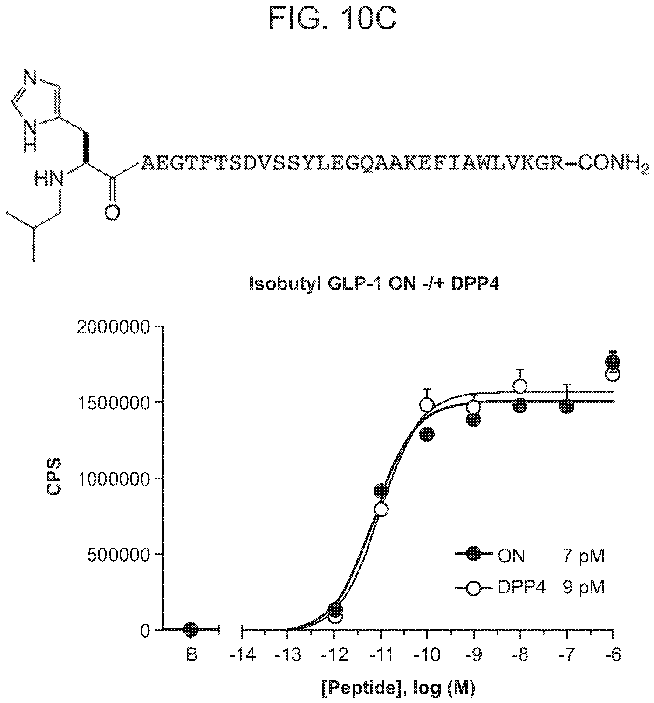

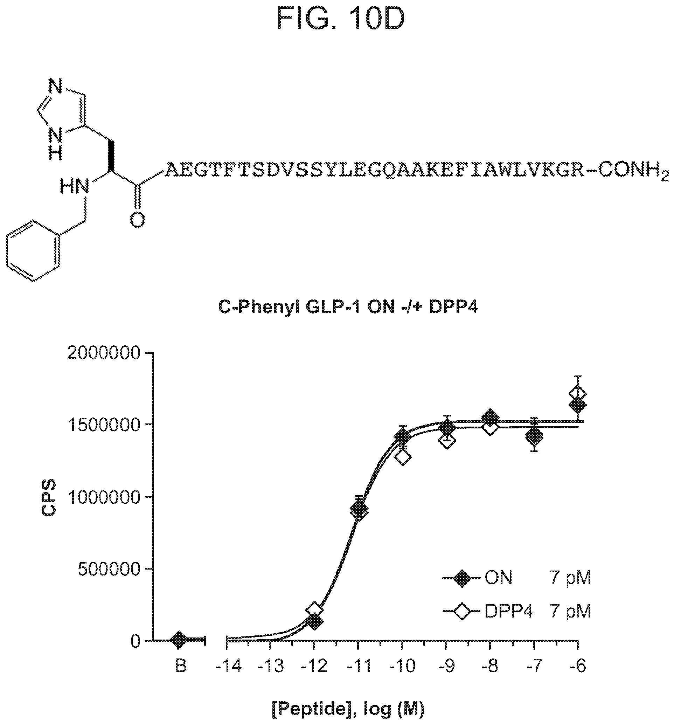

FIGs. 10A-10D provide a series of graphs that reflect stability assays (incubated overnight with or without DPP4) for acetyl GLP-1 (="G-2"; FIG. 10A), ethyl GLP-1 (="G-5"; FIG. 10B), isobutyl GLP-1 (="G-6"; FIG. 10C) and C-phenyl GLP-1 (="G-11"; FIG. 10D, nomenclature as in Fig. 6). "ON": overnight incubation. Corresponding EC50 values are indicated in pM.

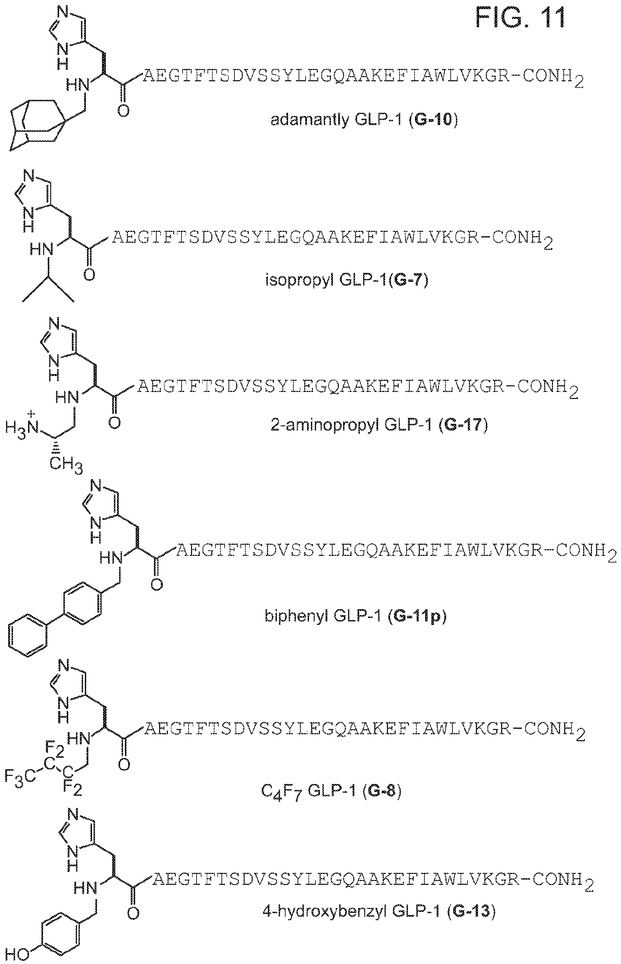

FIG. 11 provides a schematic that shows exemplary GLP-1 analogues. Nomenclature in parentheses refers to Fig. 6.

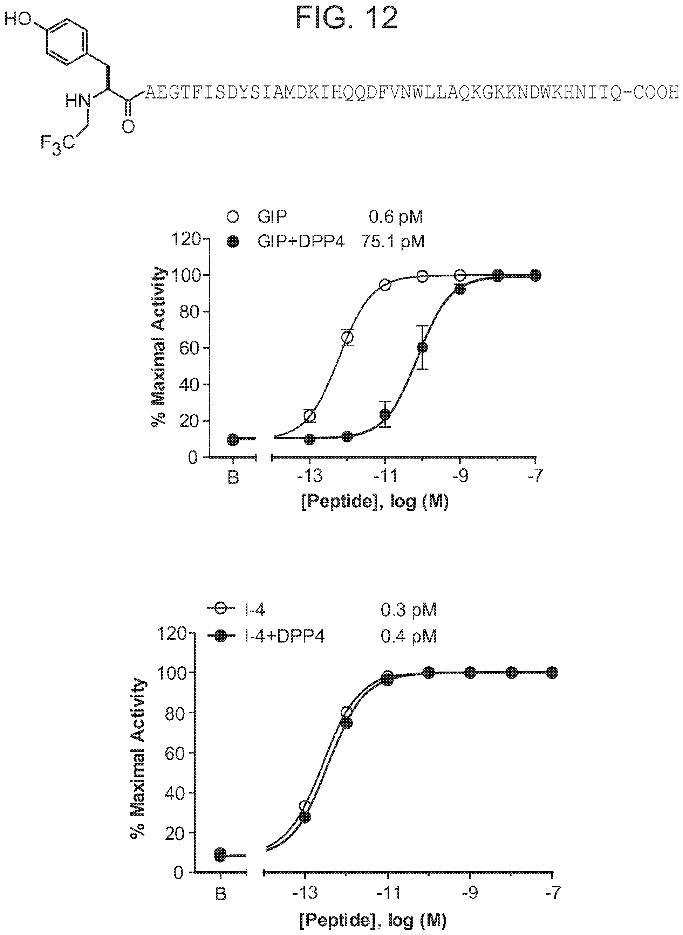

FIG. 12 provides a series of graphs showing results of stability assays (incubated with or without DPP4) for unmodified GIP vs. CHCF₃-GIP (compound 1-4). Whereas the unmodified peptide is degraded by DPP4, 1-4 is resistant to this enzyme. EC50 values are indicated in pM.

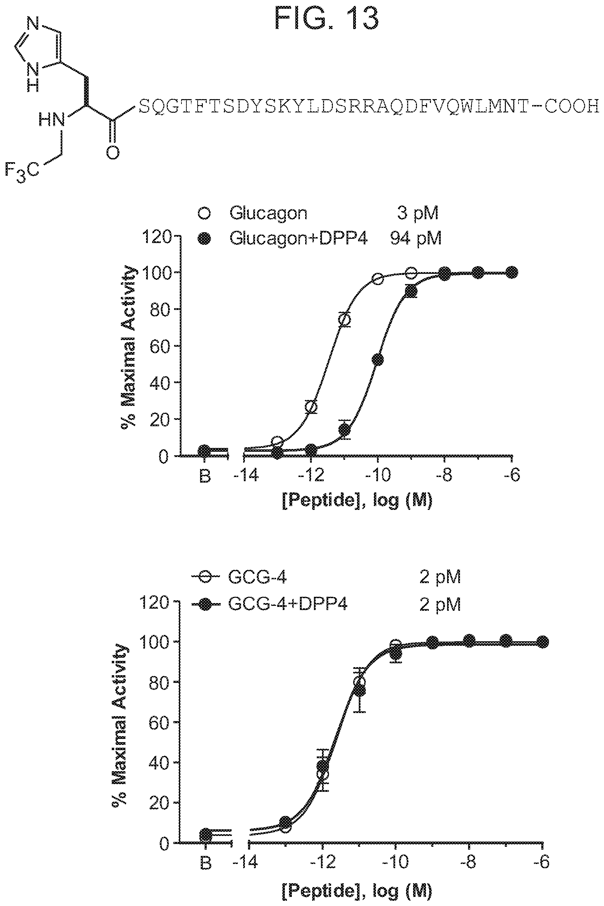

FIG. 13 provides a series of graphs showing results of stability assays (incubated with or without DPP4) for unmodified Glucagon vs. CHCF₃-glucagon (compound GCG-4). Whereas the unmodified peptide is degraded by DPP4, GCG-4 is resistant to this enzyme. EC50 values are indicated in pM.

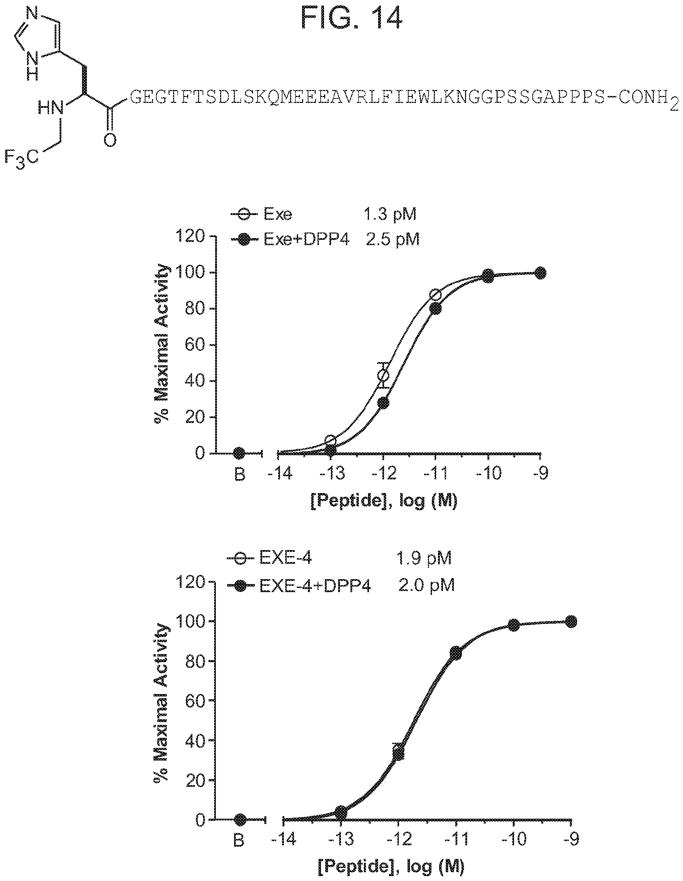

FIG. 14 provides a graph showing results of stability assays (incubated with or without DPP4) for unmodified exendin (Exe) vs. CHCF₃-exendin (compound EXE-4). Whereas unmodified exendin shows detectable sensitivity to DPP4 degradation, compound Exe-4 is completely resistant to this enzyme. EC50 values are indicated in pM.

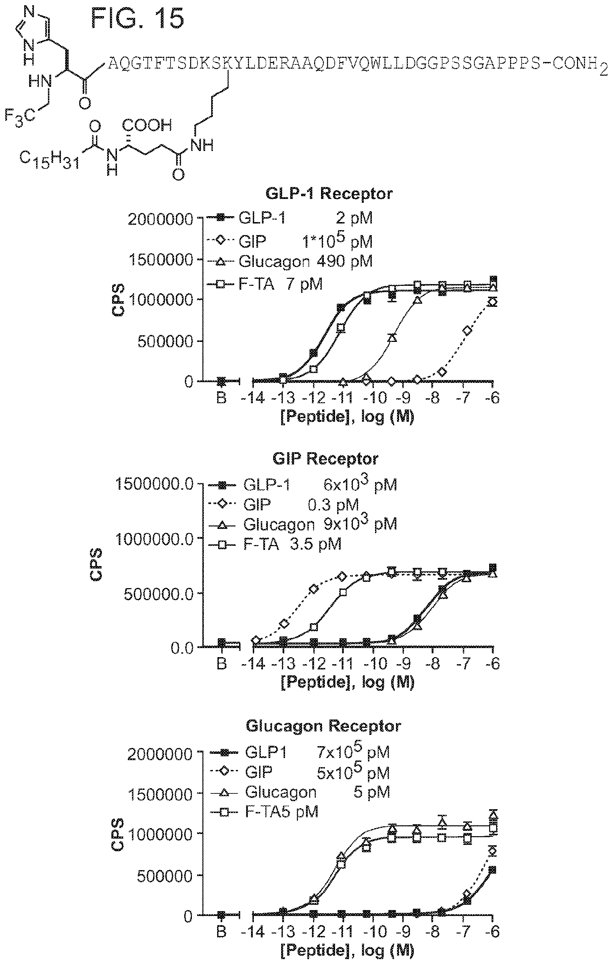

FIG. 15 provides a graph showing that a triagonist that is N-terminally decorated with CHCF₃ ("F-TA" or TA-4 in Fig. 6) co-activates the GLP-1, GIP and glucagon receptors with similar potencies. EC50 values are indicated in pM.

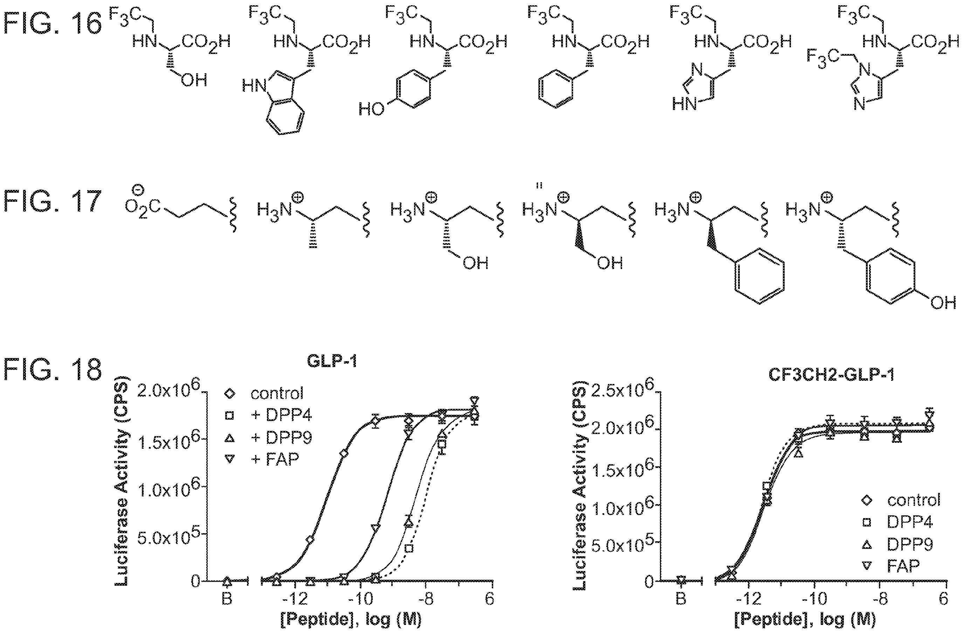

FIG. 16 provides a schematic showing modifications that are made to amino acids found at the N-terminus of secretin family peptides.
FIG. 17 provides a schematic showing the molecular structures of various charged modifications.
FIG. 18 provides a series of graphs comparing sensitivity of GLP-1 (left panel) vs. CHCF3-GLP-1 (right panel; G-4 in Fig. 6) to enzymatic degradation. Activity of these peptides, after overnight incubation without enzyme (control) or with either DPP4, DPP9, or FAP, was measured by luciferase assay in cells expressing GLP-1 receptors. In contrast to unmodified GLP-1, the G-4 derivative is resistant to enzyme induced potency loss (no rightward shift of concentration-response curve).

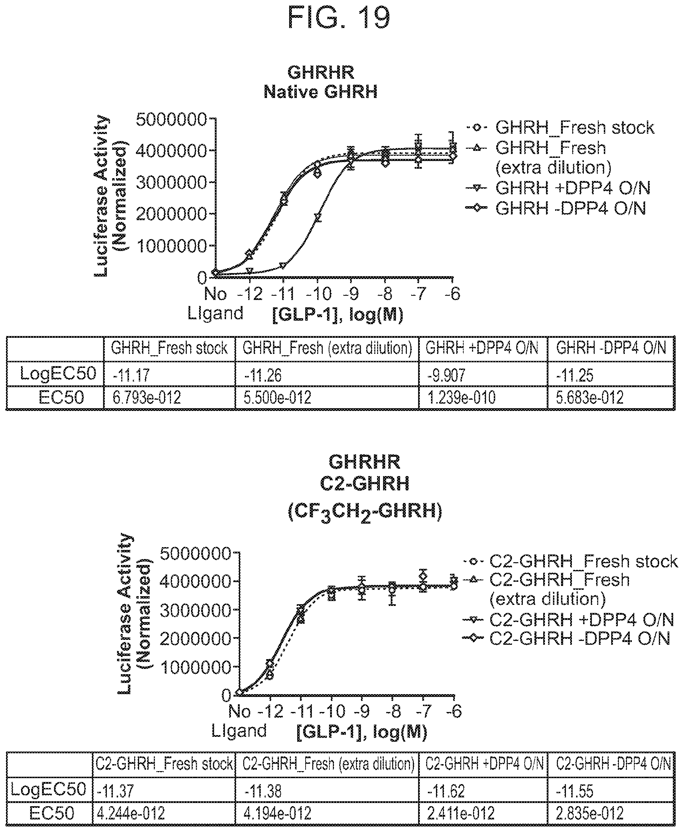

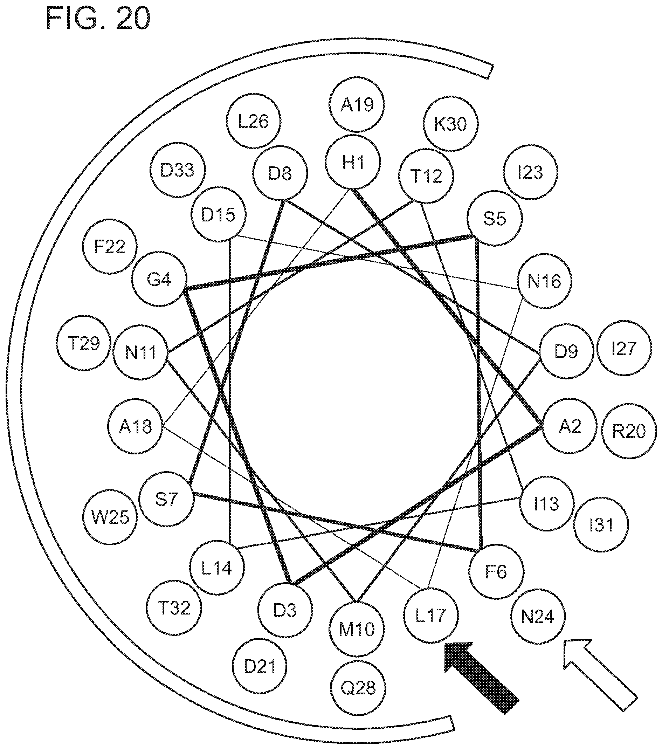

FIG. 19 provides a series of graphs comparing sensitivity of native GHRH (top panel) vs. CHCF3-GHRH ("C2-GHRH", bottom panel, compound GH29-4 in Fig. 6) to enzymatic degradation. Activity of these peptides, tested as fresh stock or after overnight (O/N) incubation either with or without DPP4, was measured by luciferase assay in cells expressing GHRH receptors (GHRHR). In contrast to native GHRH, the GH29-4 derivative is resistant to enzyme induced potency loss (no rightward shift of concentration-response curve).

### DETAILED DESCRIPTION OF THE INVENTION

The present invention generally provides modified peptides that resist proteolytic degradation while retaining the biological activity of the unmodified peptide, methods of synthesizing such modified peptide, and therapeutic methods featuring the modified peptides.

The present invention is based, at least in part, on the discovery that certain chemical modifications of polypeptides improve their resistance to proteolysis *in vitro* or *vivo,* without significantly altering their biological activity.

In certain embodiments, the chemically modified polypeptides of the invention have an improved property as compared to the corresponding chemically unmodified polypeptides, wherein the property is at least one selected from the group consisting of log P, penetration into the CNS, bioabsorption, renal clearance, efficacy (receptor stimulation), delivery/formulation, storage stability (non-protease mediated), solubility, lower propensity to aggregation/ oligomerization, resistance to Cytochrome P450 and other enzymes, ability to be used in combination with other drugs (such as with C4 agonists, melanocortin MC4 receptor, and/or serotonin), reduced immunogenicity, route of administration, and pharmacokinetic parameters.

It should be understood that the present disclosure contemplates the sequences disclosed herein, as well as sequences with at least 75%. 80%, 85%, 90%, 95%, 96%, 97%, 98%, 99% or 100% identity to the sequences disclosed herein.

As described herein, the invention provides a novel method of increasing proteolysis resistance in polypeptides using minimal chemical modification. In certain embodiments, a 2,2,2-trifluoroethyl group is chemically attached to the N-terminus amino group and/or other amino group and/or thiol group and/or thioether group in the polypeptide. The chemically modified N-terminus amino group is not charged at physiological pH, in a similar fashion to the N-terminus acetylation analogues, which are known to have good proteolytic stability. The 2,2,2- trifluoroethyl group is chemically inert. Unlike an acetyl group, the 2,2,2-trifluoroethyl group is not removed by enzymatic processes *in vivo.* Exemplary results on N-2,2,2-trifluoroethyl GLP-1 showed no loss of binding and activity in a cell-based assay, in sharp contrast to N-acetyl-GLP-1, which was about 10-50 times less active as compared to GLP-1.

In certain embodiments, the GLP-1 analogues of the invention carry low risk of hypoglycemia, have cardio- and neuroprotective effects, and stimulated GLP-1Rs in the brain to reduce appetite in the subjects to which they are administered. In other embodiments, the GLP-1 analogues of the invention promote weight loss or maintenance, or prevent weight gain, in the subjects to which they are administered.

### Definitions

Unless defined otherwise, all technical and scientific terms used herein generally have the same meaning as commonly understood by one of ordinary skill in the art to which this invention belongs. The following references provide one of skill with a general definition of many of the terms used in this invention: Singleton et al., Dictionary of Microbiology and Molecular Biology (2nd Ed. 1994); The Cambridge Dictionary of Science and Technology (Walker, Ed., 1988); The Glossary of Genetics, 5th Ed., R. Rieger, et al. (Eds.), Springer Verlag (1991); and Hale & Marham, The Harper Collins Dictionary of Biology (1991). Generally, the nomenclature used herein and the laboratory procedures in medicine, organic chemistry and polymer chemistry are those well known and commonly employed in the art.

As used herein, the articles "a" and "an" refer to one or to more than one (*i.e*., to at least one) of the grammatical object of the article. By way of example, "an element" means one element or more than one element.

As used herein, the term "about" will be understood by persons of ordinary skill in the art and will vary to some extent on the context in which it is used. As used herein when referring to a measurable value such as an amount, a temporal duration, and the like, the term "about" is meant to encompass variations of ±20% or ±10%, more preferably ±5%, even more preferably ±1%, and still more preferably ±0.1% from the specified value, as such variations are appropriate to perform the disclosed methods.

As used herein, the term "administration" means providing the compound and/or composition of the present invention to a subject by any suitable method.

As used herein, the term "Aib" refers to 2-amino isobutyric acid.

As used herein, the term "alkenyl" employed alone or in combination with other terms means, unless otherwise stated, a stable mono-unsaturated or di-unsaturated straight chain or branched chain hydrocarbon group having the stated number of carbon atoms. Examples include vinyl, propenyl (or allyl), crotyl, isopentenyl, butadienyl, 1,3-pentadienyl, 1,4-pentadienyl, and the higher homologs and isomers. A functional group representing an alkene is exemplified by -CH₂-CH=CH₂.

As used herein, the term "alkoxy" employed alone or in combination with other terms means, unless otherwise stated, an alkyl group having the designated number of carbon atoms, as defined above, connected to the rest of the molecule via an oxygen atom, such as, for example, methoxy, ethoxy, 1-propoxy, 2-propoxy (isopropoxy) and the higher homologs and isomers. Preferred are (C₁-C₃)alkoxy, such as, but not limited to, ethoxy and methoxy. As used herein, the term "alkyl" by itself or as part of another substituent means, unless otherwise stated, a straight or branched chain hydrocarbon having the number of carbon atoms designated (i.e., C₁-C₁₀ means one to ten carbon atoms) and includes straight, branched chain, or cyclic substituent groups. Examples include methyl, ethyl, propyl, isopropyl, butyl, isobutyl, tert-butyl, pentyl, neopentyl, hexyl, and cyclopropylmethyl. Most preferred is (C₁-C₆)alkyl, such as, but not limited to, ethyl, methyl, isopropyl, isobutyl, n-pentyl, n-hexyl and cyclopropylmethyl.

As used herein, the term "alkynyl" employed alone or in combination with other terms means, unless otherwise stated, a stable straight chain or branched chain hydrocarbon group with a triple carbon-carbon bond, having the stated number of carbon atoms. Non-limiting examples include ethynyl and propynyl, and the higher homologs and isomers. The term "propargylic" refers to a group exemplified by -CH₂-C≡CH. The term "homopropargylic" refers to a group exemplified by -CH₂CH₂-C≡CH. The term "substituted propargylic" refers to a group exemplified by -CR₂-C≡CR, wherein each occurrence of R is independently H, alkyl, substituted alkyl, alkenyl or substituted alkenyl, with the proviso that at least one R group is not hydrogen. The term "substituted homopropargylic" refers to a group exemplified by -CR₂CR₂-C≡CR, wherein each occurrence of R is independently H, alkyl, substituted alkyl, alkenyl or substituted alkenyl, with the proviso that at least one R group is not hydrogen. By "ameliorate" is meant decrease, suppress, attenuate, diminish, arrest, or stabilize the development or progression of a disease or disorder.

As used herein, an "amino acid" is represented by the full name thereof, by the three-letter code, as well as the one-letter code corresponding thereto, as indicated in the following table. The structure of amino acids and their abbreviations can also be found in the chemical literature, such as in Stryer, 1988, "Biochemistry", 3rd Ed., W. H. Freeman and Co., New York.

As used herein, the term "aromatic" refers to a carbocycle or heterocycle with one or more polyunsaturated rings and having aromatic character, *i.e.* having (4n+2) delocalized π (pi) electrons, where n is an integer.

As used herein, the term "aryl" or "arene" employed alone or in combination with other terms means, unless otherwise stated, a carbocyclic aromatic system containing one or more rings (typically one, two or three rings) wherein such rings may be attached together in a pendent manner, such as a biphenyl, or may be fused, such as naphthalene. Examples include phenyl, anthracyl, and naphthyl (including 1- and 2-naphthyl). Preferred are phenyl and naphthyl, most preferred is phenyl.

As used herein, the term "aryl-(C₁-C₃)alkyl" means a functional group wherein a one to three carbon alkylene chain is attached to an aryl group, e.g., -CH₂CH₂-phenyl or -CH₂-phenyl (benzyl). Preferred is aryl-CH₂- and aryl-CH(CH₃)-. The term "substituted aryl-(C₁-C₃)alkyl" means an aryl-(C₁-C₃)alkyl functional group in which the aryl group is substituted. Preferred is substituted aryl(CH₂)-. Similarly, the term "heteroaryl-(C₁-C₃)alkyl" means a functional group wherein a one to three carbon alkylene chain is attached to a heteroaryl group, e.g., -CH₂CH₂-pyridyl. Preferred is heteroaryl-(CH₂)-. The term "substituted heteroaryl-(C₁-C₃)alkyl" means a heteroaryl-(C₁-C₃)alkyl functional group in which the heteroaryl group is substituted. Preferred is substituted heteroaryl-(CH₂)-.

In one aspect, the terms "co-administered" and "co-administration" as relating to a subject refer to administering to the subject a compound and/or composition of the invention, or salt thereof, along with a compound and/or composition that may also treat any of the diseases contemplated within the invention. In one embodiment, the co-administered compounds and/or compositions are administered separately, or in any kind of combination as part of a single therapeutic approach. The co-administered compound and/or composition may be formulated in any kind of combinations as mixtures of solids and liquids under a variety of solid, gel, and liquid formulations, and as a solution.

As used herein, the terms "comprises," "comprising," "containing," "having" and the like can have the meaning ascribed to them in U.S. Patent law and can mean "includes," "including," and the like; "consisting essentially of" or "consists essentially " likewise has the meaning ascribed in U.S. Patent law and the term is open-ended, allowing for the presence of more than that which is recited so long as basic or novel characteristics of that which is recited is not changed by the presence of more than that which is recited, but excludes prior art embodiments.

As used herein, the term "composition" or "pharmaceutical composition" refers to a mixture of at least one compound useful within the invention with a pharmaceutically acceptable carrier. The pharmaceutical composition facilitates administration of the compound to a subject.

As used herein, the term "cycloalkyl" by itself or as part of another substituent means, unless otherwise stated, a monocyclic or polycyclic chain hydrocarbon having the number of carbon atoms designated (i.e., C₃-C₆ means a cyclic group comprising a ring group consisting of three to six carbon atoms) and includes straight, branched chain or cyclic substituent groups. As used herein, the term "cycloalkyl" further comprises cycloalkenyl and cycloalkynyl compounds. Examples include cyclopropyl, cyclobutyl, cyclopentyl, cyclohexyl, cycloheptyl, and cyclooctyl. An example is (C₃-C₆)cycloalkyl, such as, but not limited to, cyclopropyl, cyclobutyl, cyclopentyl and cyclohexyl. Cycloalkyl further comprises decalin, bicyclodecane, bicyclooctane, bicycloheptane, bicyclohexane, adamantyl and other polycyclic alkyl groups.

As used herein, the term "δ" refers to delta (ppm).

By "decreases" is meant a negative alteration of at least about 10%, 25%, 50%, 75%, 100%, or more.

"Detect" refers to identifying the presence, absence or amount of the analyte to be detected.

By "disease" or "disorder" is meant any condition that damages or interferes with the normal function of a cell, tissue, or organ.

As used herein, the term "Acylamino-acid-releasing enzyme" also called "acylpeptide hydrolase" refers to an enzyme that in humans is encoded by the APEH gene. The enzyme is an acylpeptide hydrolase, which catalyzes the hydrolysis of the terminal acetylated amino acid preferentially from small acetylated peptides.

As used herein, the term "DMSO" refers to dimethylsulfoxide.

As used herein, the term "DPP2" refers to dipeptidyl protease 2.

As used herein, the term "DPP4" refers to dipeptidyl protease 4.

As used herein, the term "DPP8" refers to dipeptidyl protease 8.

As used herein, the term "DDP9" refers to dipeptidyl protease 9.

As used herein, the term "FAP" refers to fibroblast activation protein, which is also known as seprase.

As used herein, the term "S9B family" refers to all S9B oligopropyl peptidases.

As used herein, the term "Vitamin D-binding protein" refers to gc-globulin. belongs to the albumin gene family, together with human serum albumin and alpha-fetoprotein. Vitamin D-binding protein is a multifunctional protein found in plasma, ascitic fluid, cerebrospinal fluid and on the surface of many cell types.

As used herein, "transferrin" refers to iron-binding blood plasma glycoproteins that control the level of free iron in biological fluids. Human transferrin is encoded by the TF gene.

As used herein, the term "GHRH" refers to Growth hormone releasing hormone. This gene encodes a member of the glucagon family of proteins. The encoded preproprotein is produced in the hypothalamus and cleaved to generate the mature factor, known as somatoliberin, which acts to stimulate growth hormone release from the pituitary gland. Variant receptors for somatoliberin have been found in several types of tumors, and antagonists of these receptors can inhibit the growth of the tumors An exemplary GHRH amino acid sequence is available at NCBI Reference No. AAB37758.1 and is provided below: 
  1 desaclqaae empnttlgcp atwdgllcwp tagsgewvtl pcpdffshfs sesgavkrdc
 61 titgwsepfp pypvacpvpl ellaeeesyf stvkiiytvg hsisivalfv aitilvalrr
121 lhcprnyvht qlfttfilka gavflkdaal fhsddtdhcs fstvlckvsv aashfatmtn
181 fswllaeavy lncllastsp ssrrafwwlv lagwglpvlf tgtwvsckla fedia
YADAIFTNSYRKVLGQLSARKLLQDIMSR-amide
YADAIFTNSYRKVLGQLSARKLLQDIMSRQQGESNQERGARARL-amide

As used herein, the term "GHRHR" refers to the receptor of Growth hormone releasing hormone.

As used herein, the term "GLP-1" refers to glucagon-like peptide-1, a neuropeptide and an incretin derived from the transcription product of the proglucagon gene. The biologically active forms of GLP-1 are: GLP-1-(7-37) and GLP-1-(7-36)NH2. Those peptides result from selective cleavage of the proglucagon molecule. GLP-1 is a potent antihyperglycemic hormone, inducing the β-cells of the pancreas to release the hormone insulin in response to rising glucose, while suppressing glucagon secretion. Exemplary GLP-1 amino acid sequences are provided below:
HAEGTFTSDVSSYLEGQAAKEFIAWLVKGRG
HAEGTFTSDVSSYLEGQAAKEFIAWLVKGRG-COOH (GLP1 7-37 acid)
HAEGTFTSDVSSYLEGQAAKEFIAWLVKGR-NH₂ (GLP-1 7-36 amide)

As used herein, the term "GLP-2" refers to glucagon-like peptide-2. GLP-2 is generated by specific post-translational proteolytic cleavage of proglucagon in a process liberates glucagon-like peptide-1 (GLP-1). Intestinal GLP-2 is co-secreted along with GLP-1 upon nutrient ingestion. GLP-2 activity can include intestinal growth, enhancement of intestinal function, reduction in bone breakdown and neuroprotection. GLP-2 and related analogs may be treatments for short bowel syndrome, Crohn's disease, osteoporosis and as adjuvant therapy during cancer chemotherapy. An exemplary GLP-2 amino acid sequence is provided below:
HADGSF SDEMNTILDNLAARDFINWLIQTKITD
As used herein, the term "PACAP" refers to Pituitary Adenylate Cyclase-Activating Polypeptide.

As used herein, the term PYY (Peptide YY3-36, and also, YY1-36) refers to peptide tyrosine or pancreatic peptide YY3-36 is a peptide that in humans is encoded by the PYY gene.

As used herein, the term "GCG" refers to Glucagon.

As used herein, the term "GIP" refers to Gastric Inhibitory Polypeptide.

As used herein, the term "SCT" refers to Secretin.

As used herein, the term "VIP" refers Vasoactive Intestinal Peptide.

By "effective amount" is meant the amount of a compound that is required to ameliorate the symptoms of a disease relative to an untreated patient. The effective amount of active compound(s) used to practice the present invention for therapeutic treatment of a disease varies depending upon the manner of administration, the age, body weight, and general health of the subject. Ultimately, the attending physician or veterinarian will decide the appropriate amount and dosage regimen. Such amount is referred to as an "effective" amount.

By "fragment" is meant a portion of a polypeptide or nucleic acid molecule. This portion contains, preferably, at least about 5%, 10%, 20%, 30%, 40%, 50%, 60%, 70%, 80%, or 90% of the entire length of the reference nucleic acid molecule or polypeptide. A fragment may contain about 5, 10, 20, 30, 40, 50, 60, 70, 80, 90, or 100, 200, 300, 400, 500, 600, 700, 800, 900, or 1,000 nucleotides or amino acids.

As used herein, the term "GLP-1" refers to glucagon-like peptide-1 or a peptide with 75% identity, preferably at least 90% identity to its sequence.

As used herein, the term "GLP-2" refers to glucagon-like peptide-2 or a peptide with 75% identity, preferably at least 90% identity to its sequence.

As used herein, the term "halo" or "halogen" employed alone or as part of another substituent means, unless otherwise stated, a fluorine, chlorine, bromine, or iodine atom, preferably, fluorine, chlorine, or bromine, more preferably, fluorine or chlorine.

As used herein, the term "heteroalkyl" by itself or in combination with another term means, unless otherwise stated, a stable straight or branched chain alkyl group consisting of the stated number of carbon atoms and one or two heteroatoms selected from the group consisting of O, N, and S, and wherein the nitrogen and sulfur atoms may be optionally oxidized and the nitrogen heteroatom may be optionally quaternized. The heteroatom(s) may be placed at any position of the heteroalkyl group, including between the rest of the heteroalkyl group and the fragment to which it is attached, as well as attached to the most distal carbon atom in the heteroalkyl group. Examples include: -O-CH₂-CH₂-CH₃, -CH₂-CH₂-CH₂-OH, -CH₂-CH₂-NH-CH₃, -CH₂-S-CH₂-CH₃, and -CH₂CH₂-S(=O)-CH₃. Up to two heteroatoms may be consecutive, such as, for example, -CH₂-NH-OCH₃, or -CH₂-CH₂-S-S-CH₃

As used herein, the term "heterocycle" or "heterocyclyl" or "heterocyclic" by itself or as part of another substituent means, unless otherwise stated, an unsubstituted or substituted, stable, mono- or multi-cyclic heterocyclic ring system that consists of carbon atoms and at least one heteroatom selected from the group consisting of N, O, and S, and wherein the nitrogen and sulfur heteroatoms may be optionally oxidized, and the nitrogen atom may be optionally quaternized. The heterocyclic system may be attached, unless otherwise stated, at any heteroatom or carbon atom that affords a stable structure. A heterocycle may be aromatic or non-aromatic in nature. In one embodiment, the heterocycle is a heteroaryl.

As used herein, the term "heteroaryl" or "heteroaromatic" refers to a heterocycle having aromatic character. A polycyclic heteroaryl may include one or more rings that are partially saturated. Examples include tetrahydroquinoline and 2,3-dihydrobenzofuryl.

Examples of non-aromatic heterocycles include monocyclic groups such as aziridine, oxirane, thiirane, azetidine, oxetane, thietane, pyrrolidine, pyrroline, imidazoline, pyrazolidine, dioxolane, sulfolane, 2,3-dihydrofuran, 2,5-dihydrofuran, tetrahydrofuran, thiophane, piperidine, 1,2,3,6-tetrahydropyridine, 1,4-dihydropyridine, piperazine, morpholine, thiomorpholine, pyran, 2,3-dihydropyran, tetrahydropyran, 1,4-dioxane, 1,3-dioxane, homopiperazine, homopiperidine, 1,3-dioxepane, 4,7-dihydro-1,3-dioxepin and hexamethyleneoxide.

Examples of heteroaryl groups include pyridyl, pyrazinyl, pyrimidinyl (such as, but not limited to, 2- and 4-pyrimidinyl), pyridazinyl, thienyl, furyl, pyrrolyl, imidazolyl, thiazolyl, oxazolyl, pyrazolyl, isothiazolyl, 1,2,3-triazolyl, 1,2,4-triazolyl, 1,3,4-triazolyl, tetrazolyl, 1,2,3-thiadiazolyl, 1,2,3-oxadiazolyl, 1,3,4-thiadiazolyl and 1,3,4-oxadiazolyl.

Examples of polycyclic heterocycles include indolyl (such as, but not limited to, 3-, 4-, 5-, 6- and 7-indolyl), indolinyl, quinolyl, tetrahydroquinolyl, isoquinolyl (such as, but not limited to, 1- and 5-isoquinolyl), 1,2,3,4-tetrahydroisoquinolyl, cinnolinyl, quinoxalinyl (such as, but not limited to, 2- and 5-quinoxalinyl), quinazolinyl, phthalazinyl, 1,8-naphthyridinyl, 1,4-benzodioxanyl, coumarin, dihydrocoumarin, 1,5-naphthyridinyl, benzofuryl (such as, but not limited to, 3-, 4-, 5-, 6- and 7-benzofuryl), 2,3-dihydrobenzofuryl, 1,2-benzisoxazolyl, benzothienyl (such as, but not limited to, 3-, 4-, 5-, 6-, and 7-benzothienyl), benzoxazolyl, benzothiazolyl (such as, but not limited to, 2-benzothiazolyl and 5-benzothiazolyl), purinyl, benzimidazolyl, benztriazolyl, thioxanthinyl, carbazolyl, carbolinyl, acridinyl, pyrrolizidinyl, and quinolizidinyl.

The aforementioned listings of heterocyclyl and heteroaryl moieties are intended to be representative and not limiting.

By "identity" is meant the amino acid or nucleic acid sequence identity between a sequence of interest and a reference sequence. Sequence identity is typically measured using sequence analysis software (for example, Sequence Analysis Software Package of the Genetics Computer Group, University of Wisconsin Biotechnology Center, 1710 University Avenue, Madison, Wis. 53705, BLAST, BESTFIT, GAP, or PILEUP/PRETTYBOX programs). Such software matches identical or similar sequences by assigning degrees of homology to various substitutions, deletions, and/or other modifications. Conservative substitutions typically include substitutions within the following groups: glycine, alanine; valine, isoleucine, leucine; aspartic acid, glutamic acid, asparagine, glutamine; serine, threonine; lysine, arginine; and phenylalanine, tyrosine. In an exemplary approach to determining the degree of identity, a BLAST program may be used, with a probability score between e⁻³ and e⁻¹⁰⁰ indicating a closely related sequence.

The term "including" is used herein to mean, and is used interchangeably with, the phrase "including but not limited to."

By "increases" is meant a positive alteration of at least about 10%, 25%, 50%, 75%, 100%, or more.

As used herein, the term "instructional material" includes a publication, a recording, a diagram, or any other medium of expression that may be used to communicate the usefulness of the compositions and methods of the invention. In some instances, the instructional material may be part of a kit useful for specifically alkylating the N-terminus amino group, other amino group, thiol group and/or thioether group of a polypeptide. The instructional material of the kit may, for example, be affixed to a container that contains the compositions of the invention or be shipped together with a container that contains the compositions. Alternatively, the instructional material may be shipped separately from the container with the intention that the recipient uses the instructional material and the compositions cooperatively. For example, the instructional material is for use of a kit; instructions for use of the compositions; or instructions for use of a formulation of the compositions.

The terms "isolated," "purified," or "biologically pure" refer to material that is free to varying degrees from components that normally accompany it as found in its native state. "Isolate" denotes a degree of separation from original source or surroundings. "Purify" denotes a degree of separation that is higher than isolation. A "purified" or "biologically pure" protein is sufficiently free of other materials such that any impurities do not materially affect the biological properties of the protein or cause other adverse consequences. That is, a nucleic acid or peptide of this invention is purified if it is substantially free of cellular material, viral material, or culture medium when produced by recombinant DNA techniques, or chemical precursors or other chemicals when chemically synthesized. Purity and homogeneity are typically determined using analytical chemistry techniques, for example, polyacrylamide gel electrophoresis or high performance liquid chromatography. The term "purified" can denote that a nucleic acid or protein gives rise to essentially one band in an electrophoretic gel. For a protein that can be subjected to modifications, for example, phosphorylation or glycosylation, different modifications may give rise to different isolated proteins, which can be separately purified.

As used herein, the phrase amide "N-terminus first internal amide bond" refers to the amide bond formed between the N-terminus amino acid of a polypeptide and the subsequent amino acid in the polypeptide (i.e., residues 1 and 2 from the N-terminus of the polypeptide).

As used herein, "naturally occurring amino acid" includes L-isomers of the twenty amino acids naturally occurring in proteins (plus cystine), as illustrated in Table 1. Unless specially indicated, all amino acids referred to in this application are in the L-form.

**Table 1. L-isomers of the twenty amino acids naturally occurring in proteins.**

| Full Name | Three-Letter Code | One-Letter Code | Full Name | Three-Letter Code | One-Letter Code |
|---|---|---|---|---|---|
| Alanine | Ala | A | Leucine | Leu | L |
| Arginine | Arg | R | Lysine | Lys | K |
| Asparagine | Asn | N | Methionine | Met | M |
| Aspartic Acid | Asp | D | Phenylalanine | Phe | F |
| Cysteine | Cys | C | Proline | Pro | P |
| Cystine | Cys-Cys | C-C | Serine | Ser | S |
| Glutamic Acid | Glu | E | Threonine | Thr | T |
| Glutamine | Gln | Q | Tryptophan | Trp | W |
| Glycine | Gly | G | Tyrosine | Tyr | Y |
| Histidine | His | H | Valine | Val | V |
| Isoleucine | Ile | I | | | |

As used herein, the terms "peptide," "polypeptide," or "protein" are used interchangeably, and refer to a compound comprised of amino acid residues covalently linked by peptide bonds. A protein or peptide must contain at least two amino acids, and no limitation is placed on the maximum number of amino acids that can comprise the sequence of a protein or peptide. Polypeptides include any peptide or protein comprising two or more amino acids joined to each other by peptide bonds. As used herein, the term refers to both short chains, which also commonly are referred to in the art as peptides, oligopeptides and oligomers, for example, and to longer chains, which generally are referred to in the art as proteins, of which there are many types. "Polypeptides" include, for example, biologically active fragments, substantially homologous polypeptides, oligopeptides, homodimers, heterodimers, variants of polypeptides, modified polypeptides, derivatives, analogues and fusion proteins, among others. The polypeptides include natural peptides, recombinant peptides, synthetic peptides or a combination thereof. A peptide that is not cyclic has a N-terminus and a C-terminus. The N-terminus has an amino group, which may be free (*i.e.,* as a NH₂ group) or appropriately protected (*e.g*., with a BOC or a Fmoc group). The C-terminus has a carboxylic group, which may be free (*i.e.,* as a COOH group) or appropriately protected (*e.g*., as a benzyl or a methyl ester). A cyclic peptide does not necessarily have free N- or C-termini, since they are covalently bonded through an amide bond to form the cyclic structure.

As used herein, the term "pharmaceutically acceptable" refers to a material, such as a carrier or diluent, which does not abrogate the biological activity or properties of the compound useful within the invention, and is relatively non-toxic, i.e., the material may be administered to a subject without causing undesirable biological effects or interacting in a deleterious manner with any of the components of the composition in which it is contained.

As used herein, the language "pharmaceutically acceptable salt" refers to a salt of the administered compounds prepared from pharmaceutically acceptable non-toxic acids or bases, including inorganic acids or bases, organic acids or bases, solvates, hydrates, or clathrates thereof. The peptides described herein may form salts with acids or bases, and such salts are included in the present invention. In one embodiment, the salts are pharmaceutically acceptable salts. The term "salts" embraces addition salts of free acids or bases that are useful within the methods of the invention. The term "pharmaceutically acceptable salt" refers to salts that possess toxicity profiles within a range that affords utility in pharmaceutical applications. Pharmaceutically unacceptable salts may nonetheless possess properties such as high crystallinity, which have utility in the practice of the present invention, such as for example utility in process of synthesis, purification or formulation of peptides useful within the methods of the invention.

Suitable pharmaceutically acceptable acid addition salts may be prepared from an inorganic acid or from an organic acid. Examples of inorganic acids include hydrochloric, hydrobromic, hydroiodic, nitric, carbonic, sulfuric (including sulfate and hydrogen sulfate), and phosphoric acids (including hydrogen phosphate and dihydrogen phosphate). Appropriate organic acids may be selected from aliphatic, cycloaliphatic, aromatic, aliphatic, heterocyclic, carboxylic and sulfonic classes of organic acids, examples of which include formic, acetic, propionic, succinic, glycolic, gluconic, lactic, malic, tartaric, citric, ascorbic, glucuronic, maleic, malonic, saccharin, fumaric, pyruvic, aspartic, glutamic, benzoic, anthranilic, 4-hydroxybenzoic, phenylacetic, mandelic, embonic (pamoic), methanesulfonic, ethanesulfonic, benzenesulfonic, pantothenic, trifluoromethanesulfonic, 2-hydroxyethanesulfonic, p-toluenesulfonic, sulfanilic, cyclohexylaminosulfonic, stearic, alginic, β-hydroxybutyric, salicylic, galactaric and galacturonic acid.

Suitable pharmaceutically acceptable base addition salts of peptides of the invention include, for example, metallic salts including alkali metal, alkaline earth metal and transition metal salts such as, for example, calcium, magnesium, potassium, sodium and zinc salts. Pharmaceutically acceptable base addition salts also include organic salts made from basic amines such as, for example, N,N'-dibenzylethylene-diamine, chloroprocaine, choline, diethanolamine, ethylenediamine, meglumine (N-methylglucamine) and procaine. All of these salts may be prepared from the corresponding peptides by reacting, for example, the appropriate acid or base with the peptide.

The term "prevent" or "preventing" or "prevention," as used herein, means avoiding or delaying the onset of symptoms associated with a disease or condition in a subject that has not developed such symptoms at the time the administering of an agent or compound commences. Disease, condition, and disorder are used interchangeably herein.

As used herein, the term "reaction condition" refers to a physical treatment, chemical reagent, or combination thereof, which is required or optionally required to promote a reaction. Non-limiting examples of reaction conditions are electromagnetic radiation, heat, a catalyst, a chemical reagent (such as, but not limited to, an acid, base, electrophile or nucleophile), and a buffer.

By "reference" is meant a standard or control condition.

A "reference sequence" is a defined sequence used as a basis for sequence comparison.

As used herein, the term "subject," "patient" or "individual" may be a human or non-human mammal or a bird. Non-human mammals include, for example, livestock and pets, such as ovine, bovine, equine, porcine, canine, feline and murine mammals. Preferably, the subject is human.

As used herein, the term "substituted" means that an atom or group of atoms has replaced hydrogen as the substituent attached to another group. Unless stated otherwise, any group recited within the invention may be substituted. ), )

The terms "treat" and "treating" and "treatment," as used herein, means reducing the frequency or severity with which symptoms of a disease or condition are experienced by a subject by virtue of administering an agent or compound to the subject.

Throughout this disclosure, various aspects of the invention may be presented in a range format. It should be understood that the description in range format is merely for convenience and brevity and should not be construed as an inflexible limitation on the scope of the invention. Accordingly, the description of a range should be considered to have specifically disclosed all the possible sub-ranges as well as individual numerical values within that range and, when appropriate, partial integers of the numerical values within ranges. Ranges provided herein are understood to be shorthand for all of the values within the range. For example, a range of 1 to 50 is understood to include any number, combination of numbers, or sub-range from the group consisting 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50.

Any compounds, compositions, or methods provided herein can be combined with one or more of any of the other compositions and methods provided herein.

Other features and advantages of the invention will be apparent from the following description of the desirable embodiments thereof, and from the claims.

### Polypeptides

In one aspect, the invention provides chemically modified polypeptides, wherein the chemical modification comprises at least one of the following: (i) at least one selected from the group consisting of an N-terminus amino group, the NH of the N-terminus first internal amide bond, other free primary amino group of the polypeptide is independently alkylatedwherein each individual alkyl is independently optionally substituted; and (ii) the NH of at least one selected from the group consisting of the N-terminus amino group and the N-terminus first internal amide bond is derivatized with X, wherein each X is independently selected from the group consisting of optionally substituted NH(C₁-C₁₆ alkyl) and is defined by the wording of the main claim referring to a chemically modified GLP-1 polypeptide, comprising an amino acid sequence having at least 75% identity to GLP-1 (H*AEGTFTS*DVS*S*YLEGQAAK*EFIAWLVK*GR), wherein at least one of the amino acid residues marked with * is alkylated with an optionally substituted C₁-C₁₆ alkyl group, wherein the N-terminal amino acid residue of the polypeptide is an N-2,2,2,-trifluoroethyl amino acid represented by the formula 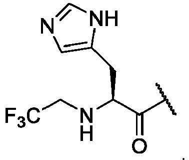 Further the invention is directed to the chemically modified polypeptide, wherein the polypeptide has 75% identity to GLP-1 (7-37) (His-Ala-Glu-Gly-Thr-Phe-Thr-Ser-Asp-Val-Ser-Ser-Tyr-Leu-Glu-Gly-Gln-Ala-Ala-Lys-Glu-Phe-Ile-Ala-Trp-Leu-Val-Lys-Gly-Arg-Gly-OH), wherein the lysine residue at position 26 (K26) has a lipid side chain (R = γ-Glu-palmitoyl); and Lys 34 (K34) is modified to an Arg residue (R34) having the structure:

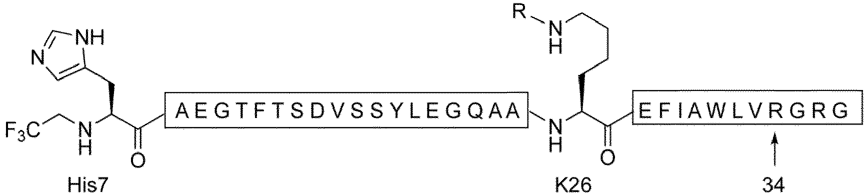

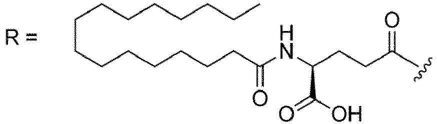

Without wishing to be limited by any theory, the group attached to the N-terminus amino group occupies a binding pocket on the receptor that is proximal to the membrane-water interface where the polypeptide binds to the receptor. It is expected that the dimensions of the binding pocket differs among receptors. In certain embodiments, for GLP-1R the binding pocket has a volume equal to or less than approximately 190 Å³, as ascertained by the fact that the N-1-adamantyl-methyl derivative of GLP-1 was found to have comparable activity to the underivatized GLP-1.

In certain embodiments, the polypeptides of the invention are more resistant to proteolytic activity by DPP4, DPP2 and/or a protease with ≥ 50% homology to DPP4 and/or DPP2 than the corresponding unmodified polypeptides. In certain embodiments, the polypeptides of the invention are more resistant to endoprotease activity than the corresponding unmodified polypeptides. In yet other embodiments, the chemically modified polypeptides have improved overall proteolytic stability in human serum over the corresponding non-chemically modified polypeptides.

The chemically modified polypeptides have improved *in vivo* half-lives over the corresponding non-chemically modified polypeptides. The chemically modified polypeptides have improved blood-brain barrier permeability over the corresponding non-chemically modified polypeptides. The chemically modified polypeptides further have improved pharmacokinetics over the corresponding non-chemically modified polypeptides.

The chemical modification contemplated in the invention is according to the claims 1. One of the alkyl groups used is -CH₂CF₃.

The polypeptides of the invention may be prepared using any methods known to those skilled in the art. In the specification it is described that the polypeptide may be prepared using standard peptide synthesis, wherein at least one of the chemically modified residues is introduced in the reaction mixture already in its chemically modified form. In other embodiments, the polypeptide may be prepared using standard peptide synthesis, wherein all of the modified residues are introduced in the reaction mixture already in their chemically modified form. In yet other embodiments, the polypeptide of the invention, or a fragment thereof, is chemically modified *in situ,* whereby the N-terminus amino group, other free amino group, thiol group (such as from a cysteine residue) and/or thioether group (such as for a methionine residue) is reacted with a reagent that promotes the alkylation of that group. The resulting chemically modified polypeptide, or fragment thereof, may be used as such in therapeutic treatments and/or further chemical reactions. Alternatively, the resulting chemically modified polypeptide, or fragment thereof, may be purified and then used as a therapeutic agent and/or submitted to further chemical reactions.

The peptides are assembled via standard automated peptide synthesis using Fmoc chemistry. Further lysine side chain amino groups are protected with an allyloxycarbonyl (alloc) group that can be selectively removed using for example PhSiH₃ and Pd(PPh₃)₄. The lysine side chain amino groups can be subsequently coupled to lipids using established chemistry.

In certain embodiments, the chemically modified polypeptides of the invention comprise one of the following sequences, wherein, in each polypeptide, at least one of the residues marked with an asterisk (*) is chemically modified as contemplated within the invention (Aib represents 2-amino isobutyric acid) and the polypeptide is optionally lipidated. GLP-1:
H*AEGTFTS*DVS*S*YLEGQAAK*EFIAWLVK*GR
   Liraglutide:
H*AEGT*FT*S*DVS*S*Y*LEGQAA-(N⁶-Palmitoylglutamyl)K*EFIAWLVRGRG

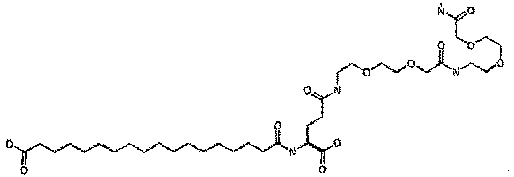

The polypeptides of the invention may possess one or more stereocenters, and each stereocenter may exist independently in either the (R) or (S) configuration. In one embodiment, polypeptides described herein are present in optically active or racemic forms. The polypeptides described herein encompass racemic, optically-active, regioisomeric and stereoisomeric forms, or combinations thereof that possess the therapeutically useful properties described herein. Preparation of optically active forms is achieved in any suitable manner, including by way of non-limiting example, by resolution of the racemic form with recrystallization techniques, synthesis from optically-active starting materials, chiral synthesis, or chromatographic separation using a chiral stationary phase. In one embodiment, a mixture of one or more isomers is utilized as the therapeutic polypeptides described herein. In another embodiment, compounds described herein contain one or more chiral centers. These compounds are prepared by any means, including stereoselective synthesis, enantioselective synthesis and/or separation of a mixture of enantiomers and/ or diastereomers. Resolution of polypeptides and isomers thereof is achieved by any means including, by way of non-limiting example, chemical processes, enzymatic processes, fractional crystallization, distillation, and chromatography.

The methods and formulations described herein include the use of N-oxides (if appropriate), crystalline forms (also known as polymorphs), solvates, amorphous phases, and/or pharmaceutically acceptable salts of polypeptides having the structure of any polypeptides of the invention, as well as metabolites and active metabolites of these polypeptides having the same type of activity. Solvates include water, ether (*e.g*., tetrahydrofuran, or methyl tert-butyl ether) or alcohol (*e.g*., ethanol) solvates, acetates and the like. In the specification the polypeptides described herein exist in solvated forms with pharmaceutically acceptable solvents such as water, and ethanol. Further the compounds described herein exist in unsolvated form.

The description discloses the polypeptides of the invention may exist as tautomers. All tautomers are included within the scope of the compounds recited herein.

It is disclosed that compounds described herein are prepared as prodrugs. A "prodrug" is an agent converted into the parent drug *in vivo.* Upon *in vivo* administration, a prodrug is chemically converted to the biologically, pharmaceutically or therapeutically active form of the polypeptide. A prodrug is enzymatically metabolized by one or more steps or processes to the biologically, pharmaceutically or therapeutically active form of the polypeptide.

Further, sites on, for example, the aromatic ring portion of polypeptides of the invention are susceptible to various metabolic reactions. Incorporation of appropriate substituents on the aromatic ring structures may reduce, minimize or eliminate this metabolic pathway. The appropriate substituent to decrease or eliminate the susceptibility of the aromatic ring to metabolic reactions is, by way of example only, a deuterium, a halogen, or an alkyl group.

Compounds described herein also include isotopically-labeled compounds wherein one or more atoms is replaced by an atom having the same atomic number, but an atomic mass or mass number different from the atomic mass or mass number usually found in nature. Examples of isotopes suitable for inclusion in the polypeptides described herein include and are not limited to ²H , ³H , ¹¹C, ¹³C, ¹⁴C, ³⁶Cl, ¹⁸F, ¹²³I, ¹²⁵I, ¹³N, ¹⁵N, ¹⁵O, ¹⁷O, ¹⁸O, ³²P, ³⁵S, ¹¹¹In, ^{99m}Tec, ⁶⁸Ga, ¹⁸F, ⁶⁴Cu, ¹²⁵I, and/or ¹³¹I. Further isotopically-labeled compounds are useful in drug and/or substrate tissue distribution studies. Further described substitution with heavier isotopes such as deuterium affords greater metabolic stability (for example, increased *in vivo* half-life or reduced dosage requirements). Further substitution with positron emitting isotopes, such as ¹¹C, ¹⁸F, ¹⁵O and ¹³N, is useful in Positron Emission Topography (PET) studies for examining substrate receptor occupancy. Isotopically-labeled polypeptides are prepared by any suitable method or by processes using an appropriate isotopically-labeled reagent in place of the non-labeled reagent otherwise employed.

It is described that the polypeptides herein are labeled by other means, including, but not limited to, the use of chromophores or fluorescent moieties, bioluminescent labels, or chemiluminescent labels.

The invention further includes a pharmaceutical composition comprising at least one polypeptide of the invention and a pharmaceutically acceptable carrier.

The pharmaceutical composition further comprises at least one additional agent that is useful to treat the diseases or disorders contemplated herein. Further it is described that the polypeptide of the invention and the additional agent are co-formulated in the composition.

### Salts

The polypeptides described herein may form salts with acids or bases, and such salts are included in the present invention. Commonly the salts are pharmaceutically acceptable salts. The term "salts" embraces addition salts of free acids or bases that are useful within the methods of the invention. The term "pharmaceutically acceptable salt" refers to salts that possess toxicity profiles within a range that affords utility in pharmaceutical applications. Pharmaceutically unacceptable salts may nonetheless possess properties such as high crystallinity, which have utility in the practice of the present invention, such as for example utility in process of synthesis, purification or formulation of polypeptides useful within the methods of the invention.

Suitable pharmaceutically acceptable acid addition salts may be prepared from an inorganic acid or from an organic acid. Examples of inorganic acids include hydrochloric, hydrobromic, hydroiodic, nitric, carbonic, sulfuric (including sulfate and hydrogen sulfate), and phosphoric acids (including hydrogen phosphate and dihydrogen phosphate). Appropriate organic acids may be selected from aliphatic, cycloaliphatic, aromatic, araliphatic, heterocyclic, carboxylic and sulfonic classes of organic acids, examples of which include formic, acetic, propionic, succinic, glycolic, gluconic, lactic, malic, tartaric, citric, ascorbic, glucuronic, maleic, malonic, saccharin, fumaric, pyruvic, aspartic, glutamic, benzoic, anthranilic, 4-hydroxybenzoic, phenylacetic, mandelic, embonic (pamoic), methanesulfonic, ethanesulfonic, benzenesulfonic, pantothenic, trifluoromethanesulfonic, 2-hydroxyethanesulfonic, p-toluenesulfonic, sulfanilic, cyclohexylaminosulfonic, stearic, alginic, β-hydroxybutyric, salicylic, galactaric and galacturonic acid.

Suitable pharmaceutically acceptable base addition salts of polypeptides of the invention include, for example, ammonium salts and metallic salts including alkali metal, alkaline earth metal and transition metal salts such as, for example, calcium, magnesium, potassium, sodium and zinc salts. Pharmaceutically acceptable base addition salts also include organic salts made from basic amines such as, for example, N,N'-dibenzylethylenediamine, chloroprocaine, choline, diethanolamine, ethylenediamine, meglumine (N-methylglucamine) and procaine. All of these salts may be prepared from the corresponding polypeptide by reacting, for example, the appropriate acid or base with the compound.

### Methods

The description includes a method of increasing proteolysis resistance of a polypeptide against a proteolytic enzyme, over the corresponding non-chemically modified polypeptide. Further the proteolytic enzyme is DPP4, DPP2 and/or other proteases with ≥ 50% homology to DPP4 and/or DPP2.

In the specification the invention includes a method of increasing serum stability of a polypeptide, over the corresponding non-chemically modified polypeptide.

The application discloses a method of improving the *in vivo* half-life of a polypeptide, over the corresponding non-chemically modified polypeptide.

Additionally the application describes a method of improving the blood-brain barrier permeability of a polypeptide, over the corresponding non-chemically modified polypeptide.

Moreover the application describes a method of improving the pharmacokinetics properties of a polypeptide over the corresponding non-chemically modified polypeptide.

In another aspect, the invention includes the chemically modified polypeptide for use in treating or preventing a disease or disorder in a subject in need thereof, wherein the use comprises administering a therapeutically effective amount of at least one polypeptide of the invention to the subject, wherein the polypeptide is optionally formulated as a pharmaceutically effective composition. It is described that the polypeptide or composition is administered to the subject by at least one route selected from oral, rectal, mucosal (*e.g*., by oral or nasal inhalation), transmucosal, topical (transdermal), and intravenous, intradermal, intramuscular, subcutaneous, intracutaneous, intrauterine, epidural and intracerebroventricular injections. The subject is further administered at least one additional agent useful for treating or preventing the disorder or disease. Additionally the subject is a mammal. Or the mammal is human. Further the disease is congenital hyperinsulinism, non-alcoholic steatohepatitis (NASH), short bowel syndrome, Alzheimer's disease, Parkinson's disease and the cessation of smoking.

### Formulations/Administration

The compositions of the present invention may contain a pharmaceutical acceptable carrier, excipient and/or diluent, and may be administered by a suitable method to a subject. The compositions of the present invention may be formulated in various forms, including oral dosage forms or sterile injectable solutions, according to any conventional method known in the art. Additionally the compositions may also be used as an inhalation-type drug delivery system. Beyond that the compositions of the invention may be formulated for injectable solutions.

The compositions may be formulated as powders, granules, tablets, capsules, suspensions, emulsions, syrup, aerosol, preparations for external application, suppositories and sterile injectable solutions. Suitable formulations known in the art are disclosed in, for example, Remington's Pharmaceutical Science (Mack Publishing Company, Easton PA). Carriers, excipients and diluents that may be contained in the composition of the present invention include lactose, dextrose, sucrose, sorbitol, mannitol, xylitol, erythritol, maltitol, starch, acacia gum, alginate, gelatin, calcium phosphate, calcium silicate, cellulose, methyl cellulose, microcrystalline cellulose, polyvinyl pyrrolidone, water, methylhydroxybenzoate, propyl hydroxylbenzoate, talc, magnesium stearate or mineral oil.

Tablets may also contain such standard ingredients as binding and granulating agents such as polyvinylpyrrolidone, disintegrants (*e.g*. swellable crosslinked polymers such as crosslinked carboxymethylcellulose), lubricating agents (*e.g.* stearates), preservatives (*e.g.* parabens), antioxidants (*e.g*., BHT), buffering agents (for example phosphate or citrate buffers), and effervescent agents such as citrate/bicarbonate mixtures. Such excipients are well known and do not need to be discussed in detail here. Capsule formulations may be of the hard gelatin or soft gelatin variety and can contain the active component in solid, semisolid, or liquid form. Gelatin capsules can be formed from animal gelatin or synthetic or plant derived equivalents thereof. The solid dosage forms (*e.g*., tablets, capsules etc.) can be coated or uncoated, but typically have a coating, for example a protective film coating (*e.g.* a wax or varnish) or a release controlling coating. The coating (*e.g.,* a Eudragit.TM. type polymer) can be designed to release the active component at a desired location within the gastro-intestinal tract. Thus, the coating can be selected so as to degrade under certain pH conditions within the gastrointestinal tract, thereby selectively release the compound in the stomach or in the ileum or duodenum. Alternatively or additionally, the coating can be used as a taste masking agent to mask unpleasant tastes such as bitter tasting drugs. The coating may contain sugar or other agents that assist in masking unpleasant tastes. Instead of, or in addition to, a coating, the antibiotic can be presented in a solid matrix comprising a release controlling agent, for example a release delaying agent which may be adapted to selectively release the compound under conditions of varying acidity or alkalinity in the gastrointestinal tract. Alternatively, the matrix material or release retarding coating can take the form of an erodible polymer (*e.g.,* a maleic anhydride polymer) which is substantially continuously eroded as the dosage form passes through the gastrointestinal tract. As a further alternative, the active compound can be formulated in a delivery system that provides osmotic control of the release of the compound. Osmotic release and other delayed release or sustained release formulations may be prepared in accordance with methods well known to those skilled in the art. The pharmaceutical formulations may be presented to a patient in "patient packs" containing an entire course of treatment in a single package, usually a blister pack. Patient packs have an advantage over traditional prescriptions, where a pharmacist divides a patient's supply of a pharmaceutical from a bulk supply, in that the patient always has access to the package insert contained in the patient pack, normally missing in patient prescriptions. The inclusion of a package insert has been shown to improve patient compliance with the physician's instructions. Each tablet, capsule, caplet, pill, etc. can be a single dose, with a dose, for example, as herein discussed, or a dose can be two or more tablets, capsules, caplets, pills, and so forth; for example if a tablet, capsule and so forth is 125 mg and the dose is 250 mg, the patient may take two tablets, capsules and the like, at each interval there is to administration.

The compositions of the present invention may be formulated with commonly used diluents or excipients, such as fillers, extenders, binders, wetting agents, disintegrants, or surfactants. Solid formulations for oral administration include tablets, pills, powders, granules, or capsules, and such solid formulations comprise, in addition to the composition, at least one excipient, for example, starch, calcium carbonate, sucrose, lactose or gelatin. In addition to simple excipients, lubricants such as magnesium stearate or talc may also be used. Liquid formulations for oral administration include suspensions, solutions, emulsions and syrup, and may contain various excipients, for example, wetting agents, flavoring agents, aromatics and preservatives, in addition to water and liquid paraffin, which are frequently used simple diluents.

Formulations for parenteral administration include sterilized aqueous solutions, non-aqueous solutions, suspensions, emulsions, freeze-dried preparations, and suppositories. As non-aqueous solvents or suspending agents, propylene glycol, polyethylene glycol, plant oils such as olive oil, or injectable esters such as ethyl oleate may be used. As the base of the suppositories, witepsol, Macrogol, Tween 61, cacao butter, laurin fat, or glycerogelatin may be used.

The dose of the pharmaceutical compositions of the present invention varies depending on the patient's condition and weight, the severity of the disease, the type of drug, and the route and period of administration and may be suitably selected by those skilled in the art. For certain effects, the pharmaceutical composition of the present invention may be administered at a dose of 0.01-100 mg/kg/day. The administration may be anywhere from 1 to 4 times daily, *e.g*., once, twice, three times or four times daily. The maximum amount administered in a 24 hour period may be up to 1500 mg. The administration may be over a course of 2 to 30 days, *e.g*., 3 to 21 days, such as 7, 10 or 14 days. The skilled person can adjust dosing depending on the subject's body weight and overall health condition and the purpose for administering the antibiotic. Repeated courses of treatment may be pursued depending on the response obtained.

The compositions of the present invention may be administered to a subject by various routes. All modes of administration are contemplated, for example, orally, rectally, mucosally (*e.g*., by oral or nasal inhalation), transmucosally, topically (transdermal), or by intravenous, intradermal, intramuscular, subcutaneous, intracutaneous, intrauterine, epidural or intracerebroventricular injection.

Unless otherwise indicated, all numbers expressing quantities of ingredients, properties such as molecular weight, reaction conditions, and so forth used in the specification and claims are to be understood as being modified in all instances by the term "about." Accordingly, unless indicated to the contrary, the numerical parameters set forth in the following specification and attached claims are approximations that may vary depending upon the desired properties sought to be obtained by the present invention. At the very least, and not as an attempt to limit the application of the doctrine of equivalents to the scope of the claims, each numerical parameter should at least be construed in light of the number of reported significant digits and by applying ordinary rounding techniques.

Notwithstanding that the numerical ranges and parameters setting forth the broad scope of the invention are approximations, the numerical values set forth in the specific examples are reported as precisely as possible. Any numerical value, however, inherently contain certain errors necessarily resulting from the standard deviation found in their respective testing measurements.

It is to be understood that wherever values and ranges are provided herein, all values and ranges encompassed by these values and ranges, are meant to be encompassed within the scope of the present invention. Moreover, all values that fall within these ranges, as well as the upper or lower limits of a range of values, are also contemplated by the present application.

### EXAMPLES

The invention is now described with reference to the following Examples. These Examples are provided for the purpose of illustration only, and the invention is not limited to these Examples, but rather encompasses all variations that are evident as a result of the teachings provided herein.

Unless noted otherwise, the starting materials for the synthesis described herein were obtained from commercial sources or known synthetic procedures and were used without further purification.

### Animals:

20-24 weeks old C57blk6J wild type male mice are used to investigate the acute effect of GLP1 analogues on improving glucose metabolism. Mice are fed with chow diet and housed individually for the length of study (up to 8 weeks). Mice are treated with GLP-1 analogues and submitted to a glucose tolerance test to assess treatment outcome.

To detect statistically significant differences in the parameters to be measured, 10 mice are used per experimental condition and end point. The male mice are housed individually to avoid fighting during handling and study. Two cohorts of mice are used: a first cohort of 40 mice (4 experimental groups of 10 mice each) to determine dosing and time interval for action of reference product liraglutide, and a second cohort of 50 mice (5 experimental groups of 10 mice each) to determine the properties of new GLP-1 analogues.

A total of 130 mice are used for the GLP2 studies. Five-week old male C57BL/6J mice are allowed to acclimatize for one week prior to any experimentation. The animals are administered with GLP-2 analogues (experimental groups) or GATTEX^{®} (control group). 4 doses of each compound are tested. Mice are euthanased at two time points after initiating the injections (6 or 10 days) to collect intestinal segments for histomorphometry analysis. Additional groups of animals (one group per each compound) undergo PET/CT imaging at various time points after the initiation of the injections. The animals are housed and maintained on a 12-h light-dark cycle (lights on at 07:00) in a ventilated facility with a steady ambient temperature ranging from 19 to 22 °C and humidity ranging from 40 to 60%.

### Assay for activity and stability:

Cellular assays for receptor binding and intracellular signaling were carried out with the compounds of the invention. Briefly, HEK 293 cells were transiently transfected with cDNAs encoding (i) GLP-1R (or the empty expression vector), (ii) the CRE₆ₓ-LUC reporter gene (which senses cAMP production triggered by receptor stimulation), and (iii) β-galactosidase (as a control for transfection efficiency). After transfection for 24 h, cells were incubated with a peptide at various concentrations (or control/blank) in serum-free medium for 6 h. The cells were then lysed, SteadyLite reagent (PerkinElmer) is added, and luciferase activity is measured in a TopCount NXT HTS plate luminometer. After further incubation with a chromogenic substrate (2-nitrophenyl β-D-galactopyranoside), optical density at 420 nm is determined by spectrophotometry, as a control to correct for interwell transfection variability. Sigmoidal concentration-response curves of GLP-1R ligand activity is calculated to determine potency.

Plotting concentration versus the luciferase activity yielded quantitative parameters for combined binding (potency) and signaling (efficacy) in a single step. Cells were stimulated with peptides, DPP4-treated and controls, for 4 hours. DPP4 treated peptides: 10 µl of 0.26mM peptide were incubated with 2 µL DPP4 in buffer ± DMSO for 18hrs. Control peptides: 10 µl of 0.26mM peptide was added to buffer ± DMSO. The final DMSO concentration was the same for all peptides and all peptides were diluted to 0.26 mM concentrations for the DPP4 assay.

### Detection of peptide inactivation by DPP4:

Compounds are incubated overnight at 37 °C in the presence of recombinant DPP4 or vehicle (TRIS buffer). The samples are then assessed in GLP-1R agonist assays. As DPP4 induced N-terminal cleavage of peptides result in virtually complete loss of function, the decrease in intact peptide is reflected in a corresponding potency loss (DPP4 vs. vehicle treated; *e.g.* 10-fold potency loss indicates 90% degradation). An example of this analysis is illustrated in FIG. 8. Complementary assessment is made by ESI LC-MS (as in FIG. 6) to detect potential minor degrees of DPP4 mediated hydrolysis that are difficult to detect by comparison of potency in functional assays.

### Example 1: Modified Glucagon Like Peptide 1 (GLP-1)

As demonstrated herein, the invention provides a method of ameliorating, preventing or minimizing the proteolysis instability problem that is ubiquitous to peptides. Such methods comprise the preparation of novel polypeptides that are chemically modified.

In the invention described herein, the modification comprises covalently attaching at least one 2,2,2-trifluoroethyl group to the N-terminal amino group, other free amino group, thiol group and/or thioether group in a polypeptide. Such modification may be achieved using various synthetic groups. In a non-limiting example, this chemical derivatization may be performed using a rapid and efficient fluoroalkylation reaction, which is demonstrated with two analogues of Glucagon Like Peptide 1 (GLP-1).

GLP-1 synthesized by solid phase peptide synthesis was derivatized while resinbound to yield the desired trifluoroethyl analogue, with equivalent ease and speed as the predictable and well-practiced acetylation of peptides. The N-acetyl analogue of GLP-1 was prepared as a control, besides GLP-1 itself. A bis-trifluoroethyl analogue of GLP-1 was also obtained. HPLC traces of native and modified GLP-1 variants are shown at FIG. 1. The chemistry involved in this modification is illustrated in FIG. 2.

Two exemplary chemically modified peptides were synthesized, along with corresponding control polypeptides, using a single automated synthesis. The sequence HAEGTFTSDVSSYLEGQAAKEFIAWLVKGR-NH₂ (GLP-1 7-36 amide) was prepared via solid-phase Fmoc-chemistry in an automated synthesizer. The solid support was MBHA-Rink amide resin, loading 0.35 mmol/gram. From 0.1 meq of resin (280 mg) were obtained 629 mg of resin carrying the crude GLP-1.

Three 100-mg portions of derivatized resin, carrying in theory 0.016 mmol of GLP-1 each, were treated as follows:
**Portion 1:** Deprotection with 20% piperidine in dimethylformamide (DMF); washing with DMF; washing with dichloromethane (DCM); cleavage with trifluoroacetic acid (TFA), triisopropylsilane (TIPS) and water (95/2.5/2.5); evaporation to small volume; and precipitation in ether gave after lyophilization 26 mg of crude GLP-1.
**Portion 2:** Deprotection with 20% piperidine in DMF; washing with DMF; treatment with acetic anhydride (1.5 mL) and diisopropylethyl amine (DIEA, 0.15 mL) in DMF (10 mL); washing with DMF; washing with DCM; cleavage with TFA/TIPS/water 95/2.5/2.5; evaporation to small volume; and precipitation in ether gave after lyophilization 21 mg of crude N-Acetyl-GLP-1.
**Portion 3:** Deprotection with 20% piperidine in DMF; washing with DMF; washing with DCM; adding phenyl(trifluoroethyl)iodonium triflimide **1a** (9 mg, 0.016 mmol, in DCM, 2 mL) for 3 minutes; then adding collidine (10 mg, 0.08 mmol, in DCM, 4 mL) for 5 min; then washing with DMF; washing with DCM; cleavage with TFA/TIPS/water 95/2.5/2.5; evaporation to small volume; and precipitation in ether gave after lyophilization 30 mg of crude N-trifluoroethyl-GLP-1. This crude material contained 2,2,2-trifluoroethyl-GLP-1 (FIG. 4) as a major component and (bis-2,2,2-trifluoroethyl)-GLP-l as a minor component (according to ESI/MS).

Each crude material was purified by HPLC. Analytical HPLC showed purities in excess of 96%. Retention times (Vydac 218TP104 C-18 column, 35-55% solvent B in solvent A over 20 minutes) were 9.6 min (GLP-1), 10.9 min (N-acetyl-GLP-1), 11.6 min (N-(2,2,2-trifluoroethyl)-GLP-l), and 13.4 min ((Nα,Nπ-Bis-2,2,2-trifluoroethyl)-GLP-1). ESI/MS data were consistent with pure compounds. Solvent A = 99% H2O, 1% CH3CN, 0.1% TFA; Solvent B = 90% CH3CN, 10% H2O, 0.07% TFA.

### Example 2: Sensitivity to DPP4

Compounds were incubated overnight at 37 °C in the presence of recombinant DPP4 or vehicle (TRIS buffer, pH 8.0). The samples were then assessed in GLP1R agonist assays as described elsewhere herein. As DPP4 induced *N*-terminal cleavage of peptides result in virtually complete loss of function, the decrease in intact peptide was reflected in a corresponding potency loss (DPP4 vs. vehicle treated; *e.g*., 10-fold potency loss indicates 90% degradation). Non-limiting examples are illustrated in FIG. 3. Complementary assessment can be made by ESI LC-MS to detect potential minor degrees of DPP4 mediated hydrolysis that may be difficult to detect by comparison of potency in functional assays.

**Table 2: Agonist potency and sensitivity to DPP4-mediated hydrolysis.**

| **Polypeptide** | **N-Terminus amino modifications** | **Receptor** | **EC₅₀ (pM)** | **DPP4 cleavage** |
|---|---|---|---|---|
| GLP-1 | - | GLP-1R | 2.2 | +++ |
| | 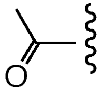 | GLP-1R | 99.1 | - |
| | 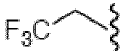 | GLP-1R | 1.3 | - |
| | 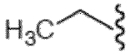 | GLP-1R | 6.4 | - |
| | 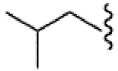 | GLP-1R | 3.7 | - |
| | 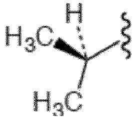 | GLP-1R | 4.1 | - |
| | 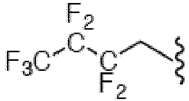 | GLP-1R | 1.0 | - |
| | 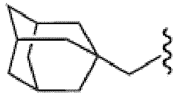 | GLP-1R | 3.9 | - |
| | 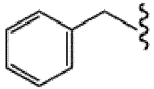 | GLP-1R | 4.4 | - |
| | 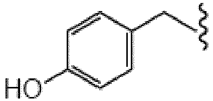 | GLP-1R | 1.0 | n.d. |
| | 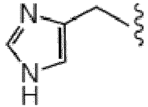 | GLP-1R | 2.7 | n.d. |
| | 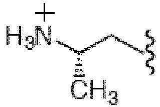 | GLP-1R | 2.6 | - |
| | 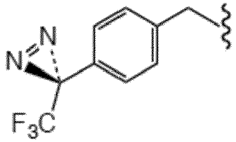 | GLP-1R | 123 | n.d. |
| | 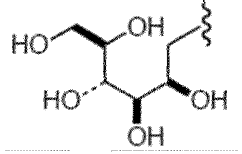 | GLP-1R | 3.2 × 10³ | - |
| liraglutide | - | GLP-1R | 1.2 | ++ |
| | 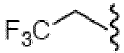 | GLP-1R | 1.7 | - |
| exenatide | - | GLP-1R | 1.1 | + |
| | 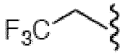 | GLP-1R | 1.2 | - |
| GIP | - | GIPR | 0.9 | +++ |
| | 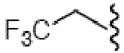 | GIPR | 0.6 | - |

| | | | | |
|---|---|---|---|---|
| GLP-1 corresponds to GLP-1(7-36); GIP corresponds to GIP(1-42). n.d. = not determined | | | | |

Non-limiting constructs contemplated within the invention are illustrated in Scheme 1 (FIG. 6), wherein X represents a chemical modification contemplated within the invention: 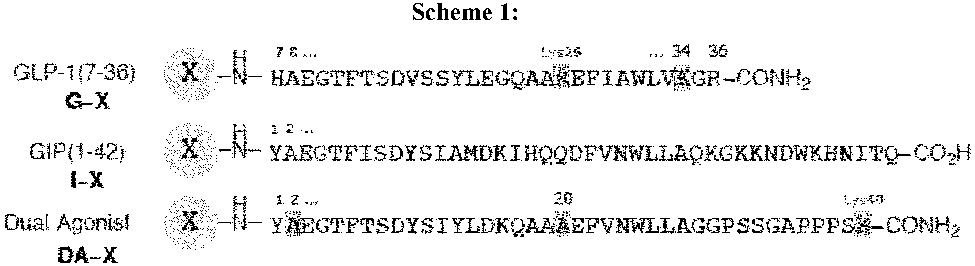
GLP-1(7-36) amide: X = H;
GIP(1-42) acid: X = H;
dual agonist: X = H, Lys40 is acyl C16 derivatized, Ala 2 and/or Ala 20 are independently optionally Aib;
liraglutide = GLP(7-37) acid with residue 34 = Arg, residue 37 = glycine, and Lys26 is acylated with γ-Glu-C16 acid

### Assessing the role of size and other molecular properties of residue modification:

In order to establish the molecular parameters that best complement the receptor binding site (that may be elastic depending on the ligand), constructs are tested for various attributes such as size, hydrophobicity, charge, polarizability, stereochemistry and electronegativity. While the predicted p*K*a values of appendages that were tolerated in GLP-1 indicate that the positive charge is not essential, the native ligand is partially charged at physiological pH. Assuming that the attached groups are accommodated in the binding pocket, the electron withdrawing and donating nature of the substituent at the 4-position on the aryl ring of the *N*-benzyl derivative is varied to assess electronic factors.

### Photoaffinity probes for identifying the site of residue interaction with the receptor:

The N-terminal regions of GLP-1 and related peptides that interact directly with their cognate receptors have eluded structural characterization using either crystallization or cross-linking approaches. A limitation to the latter is the lack of suitable photoaffinity probes with modifications of His7 that are compatible with GLP-1 binding to its receptor. To generate such tools, modest structural perturbation to the already characterized ligands that exhibit potencies and efficacies similar to the native ligand is utilized. Thus, the paratrifluoromethyldiazirine benzyl derivative was prepared. This compound, while maintaining full agonist efficacy, was 123-fold less potent than GLP-1, presumably due to the steric bulk at the 4-position of the aryl group. The para-azido benzyl and the methyldiazirine ethyl derivatives are further prepared and studied. The photoactive groups diazirine and phenylazide are selected due to their spectral characteristics and their ability to rapidly insert into C-H bonds in close vicinity once reactive carbene or nitrene are generated. In certain embodiments, the polypeptide constructs are additionally equipped with another affinity tag (*e.g*., biotin) coupled to Lys26, a site where attachments (*e.g*., lipidation in liraglutide) are well tolerated. After incubation with isolated cell membranes containing GLP1R and photocross-linking, the receptor-ligand complex is affinity purified, followed by digestion with proteases. The site of labeling is then identified by mass analysis, to be carried out either on gel by MALDI MS or by LC ESI-MS.

The data presented herein demonstrates that a diverse range of modifications are accepted at the N-terminus of the ligand without compromising receptor agonism. At the same time, these alterations provide complete protection against hydrolytic action mediated by DPP4. Together, these findings open new opportunities to elucidate the structural determinants of receptor activation that may be applicable to other class B GPCRs.

### Example 3: DPP4-resistant analogues

Resistant analogs are shown at Fig. 6-10, 12,13, 14, 18, 19 and 21. DPP4-insensitive analogues of liraglutide, a palmitoylated form of GLP-1, are generated by derivatizing the His7 residue. Palmitoylation promotes peptide multimerization and enhances reversible serum albumin binding as mechanisms to partially protect liraglutide. The potential synergistic effects of His7 N-alkylation and lipidation in liraglutide are investigated. His7 modifications in a GLP-1 analogue with an alternative lipidation (compound "A6"), which may also enhance albumin binding, are also explored.

In the specification it is described that the presently contemplated modifications of the terminal His7 in GLP-1 and analogues selectively abolish cleavage by DPP4 without compromising agonist activity. Further, the presently contemplated modifications of the terminal His7 in GLP-1 and analogues essentially maintain the native non-helical N-terminal conformation of GLP-1 and interactions with the receptor that are critical for affinity and agonist function. Additionally, the presently contemplated modifications of the terminal His7 in GLP-1 and analogues allow for exploring details of the GLP-1R ligand binding pocket vs. recognition by DPP4, and also enable fine-tuning the polypeptide to facilitate access to different biological compartments (*e.g*., mucosal uptake after oral or nasal application; crossing the blood brain barrier to potentially optimize drug-induced weight loss as future directions).

As demonstrated herein, liraglutide is in fact quite extensively inactivated by DPP4. Assessment of active drug levels in serum by an improved sensitive bioassay presents a significant technological advance vs. conventional peptide-specific RIAs / ELISAs (as typically used in the industry, *e.g*. during drug development of liraglutide and exenatide). Whereas conventional sandwich immunoassays rely on specific antibodies that concomitantly detect a peptide's intact N- and C-termini, the present approach is universally applicable to new potent agonists regardless of structural modification.

### In vitro Studies

As part of the structure-function analyses with native GLP-1, seven analogues with differential chemical attributes are engineered. His7 modifications are introduced in either liraglutide or an analogue with an alternative lipid moiety. Cell based assays are used to assess the potency and efficacy of these peptides at the GLP-1R. Parallel studies are used to quantify inactivation after prolonged exposure to DPP4 *in vitro.* Direct comparison is made with liraglutide. Potent/stable analogues may be selected for *in vivo* study.

*In vitro* experiments confirmed that unmodified GLP-1 is highly susceptible to DPP4 degradation. After overnight incubation with the enzyme, apparent potency was >500-fold reduced, indicating more than 99.8% of the peptide was degraded/inactivated. Liraglutide, a lipidated GLP-1 analogue for use as a diabetes drug, is reported to be more DPP4 resistant but is still degraded by this enzyme *in vivo.* In fact, liraglutide was shown to suffer a >50-fold potency loss after incubation with DPP4, indicating that more than 98% of the peptide was degraded.

His7 at the N-terminus of GLP-1 plays a crucial role in GLP-1R activation, but at the same time enables peptide recognition and degradation by DPP4. Modifications of His7 have been considered impractical for stabilizing GLP-1. Prior attempts to substitute or modify His7, *e.g.* by acetylation, provide DPP4 resistance but also lead to a major loss of agonist potency (FIG. 8, 9, and 9a). The present functional assays showed that this analogue had stability against DPP4, coupled with a ~50-fold potency loss of GLP-1b.

However, as demonstrated herein, the present modifications of selectively enhance the stability of GLP-1 without disrupting its agonist function. Trifluoroethyl, isobutyl, or benzyl analogues caused barely detectable, and non-significant potency changes while conferring virtually complete DPP4 resistance.

Further, trifluoroethylation did not affect liraglutide potency. Furthermore, this analogue showed complete resistance to DPP4 degradation. The remaining analogues contemplated within the invention may be evaluated for agonist activity and enzymatic stability using similar assays. Such analogues include liraglutide analogue "A6", which includes an alternative lipid moiety in position R2 that confers markedly higher albumin binding (lipid *l*₂). A6 is cleared as quickly as liraglutide, possibly due to remaining degradation of A6 by DPP4.

Peptides are assembled via standard automated peptide synthesis using Fmoc chemistry. Lys26 is side chain-protected with an allyloxycarbonyl (alloc) group that can be selectively removed using PhSiH₃ and Pd(PPh₃)₄, and subsequently coupled to *l*₁ or *l*₂ in position R² (FIG. 7) using established chemistry. The N-terminal modifications in position R¹ is introduced via reductive amination on the solid phase, using the corresponding aldehydes and NaBH₄. In the case of the trifluoroethyl containing compounds (GLP-1c, MG1 and MG4; FIG. 7), the histidine derivative is independently synthesized. The constructs are cleaved from the resin using CF₃CO₂H:TIPS:H2O (95:2.5:2.5), purified using reversed-phase HPLC, and analyzed using ESI and MALDI-MS.

### In vivo Studies

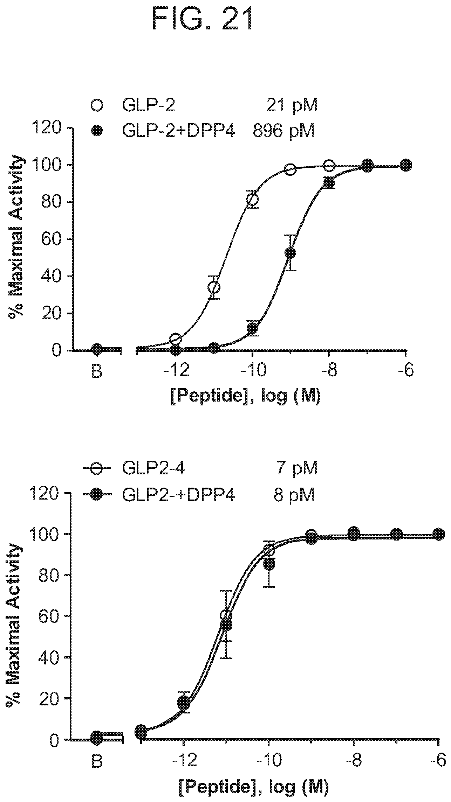

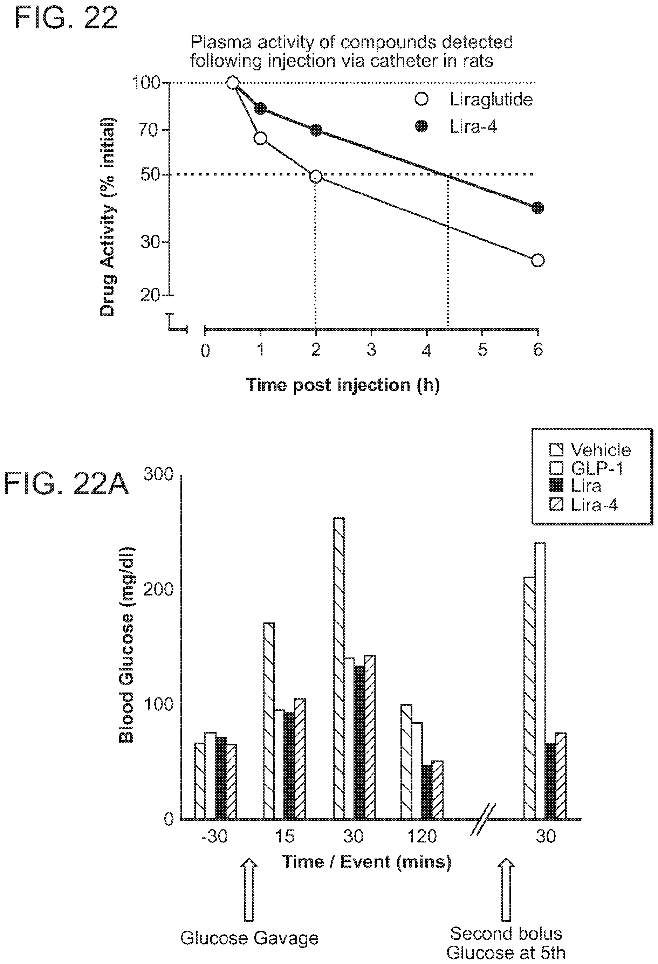

In one aspect, attenuation of blood glucose excursion by GLP-1 analogues was analyzed (FIG. 22A). FIG. 21 provides a series of graphs showing that native GLP-2 is degraded by DPP4 whereas a trifluoroethyl decorated analog (GLP2-4) is completely resistant to degradation. Peptides were incubated with recombinant DPP4. Serial dilutions of GLP-2 and GLP2-4 were then applied to HEK293 cells, which had been transfected with cDNA encoding human GLP-2R and a cAMP-responsive reporter gene. DPP4 induced a >40-fold loss in GLP-2 potency, reflecting that >97% of peptide was degraded. In contrast, no potency loss was noted with GLP2-4. N=4, mean+SEM.

Fig. 22A provides a graph comparing the time-dependent decrease of plasma drug activity after a bolus injection of either liraglutide or a CHCF3-decorated derivative, Lira-4. Rats received a bolus injection of either drug via central catheter, followed by serial blood draws after indicated intervals and measurement of plasma drug activity by bioassay of receptor agonism. Potency of each peptide immediately following injection was defined as 100%. Plasma survival of liraglutide is extended by the CHCF3 modification of Lira-4.

Fig. 22B demonstrates sustained hypoglycemic activity of Lira-4 following an oral glucose tolerance test. Mice received a s.c. injection of either vehicle, G-4, Liraglutide, or Lira-4 (represented from left to right in each group of bars). Half an hour and 5.5 hours later, two sequential oral glucose loads were applied by gavage. Blood glucose levels were measured just before drug injection (-30 minutes) and at indicated time intervals after the glucose loads. A single injection of compound G-4 attenuated glycemic excursion after the first glucose load, and still remained active to attenuate a second glucose challenge several hours later.

### Example 5: Modifications to the N-terminus of GLP-1.

The following experiments address an ongoing challenge in the design of therapeutics based on agonism at the GLP-1R: *combining* resistance to DPP4 with retention of potency and efficacy during receptor activation (See Table 3). The data established that the GLP-1/GLP-1R system is tolerant of a wide range of derivatives that exhibit both of the desired properties. First explore the structural space available for creation of GLP-1 analogs will be explored. A two-pronged approach is used to narrow the potentially large landscape of possible candidates (*vide infra*). The chemical decorations of the *α*-amine of His7, situated at the N-terminus of GLP-1, span a range of functionalities and sizes. From known SAR in the literature and the data presented herein, there are a few known requirements: (i) the presence of the methyl imidazole side chain of His7 is critical; (ii) *N*-acyl (including *N*-acetyl) derivatives result in poor potency and efficacy (iii) large polar appendages on the nitrogen are not tolerated (e.g. *N*-mannitol or *N*-glucitol). Nevertheless, the data demonstrate that a range of modifications, including charged groups at physiological pH are allowed. Here, the working model envisions a binding pocket on the receptor, proximal to the membrane-water interface that accommodates the chemical alterations made to histidine. The exact dimensions of this pocket and the precise functional makeup remain to be defined, however a GLP-1R binding box with a volume of approximate 240 Å³ or slightly larger can be assumed (based on the activity of the *N*-methyl adamantyl derivative G-10). To test this assumption, and to explore the nature of putative receptor-ligand interactions within this binding box, a library of 50 GLP-1 derivatives is prepared to explore criteria of size, hydrophobicity, charge, polarity, stereochemistry (G17-21) and polarizability. Exemplary GLP-1 derivatives are shown in FIG. 6A.

### Example 6: Chemical synthesis of conjugates.

Peptides will be assembled via standard automated peptide synthesis using Fmoc chemistry. Lys26 in GLP-1 will be side chain-protected with an allyloxycarbonyl (alloc) group that can be selectively removed using PhSiH₃ and Pd(PPh₃)₄, and if required subsequently coupled to lipids using established chemistry. The *N*-terminal modifications ("X", FIG. 6A and 6B) will be introduced via reductive amination on the solid phase, using the corresponding aldehydes and NaBH₄ (89). In the case of the compounds containing fluoroalkyl and azido functionalities, the histidine derivative is independently synthesized using previously reported procedures (90-95). The constructs will be cleaved from the resin using CF₃CO₂H:TIPS:H₂O (95:2.5:2.5), purified using reversed-phase HPLC, and analyzed using ESI and MALDI-MS.

### Example 7: Photoaffinity probes for identifying the site of His7 interaction in GLP-1 with the receptor.

The *N*-terminal regions of GLP-1 and related peptides that interact directly with their cognate receptors have eluded structural characterization using crystallization or cross-linking approaches. A critical limitation to the latter is the lack of suitable photoaffinity probes with modifications of His7 that are compatible with GLP-1 binding to its receptor. To generate such tools, structural perturbations will be made to the ligands that exhibit potencies and efficacies similar to the native ligand. The compound **G-22** while maintaining full efficacy showed markedly reduced potency compared to unmodified GLP-1, presumably due to the steric bulk at the 4-position of the aryl group (FIG. 6A). Agonist potency was conserved in photoprobe **G-30** suggesting that the latter will recapitulate the interaction of unmodified GLP-1 with the receptor. To facilitate detection in cross-linking studies, further a biotin moiety may be attached to residue K26 in GLP-1. The data has shown that this addition does not affect GLP-1 affinity, consistent with the fact that the same residue is also used for acylation of liraglutide (FIG. 6A).

### Example 8: Assessing the role of size and other molecular properties of His7.

In order to establish the molecular parameters that best complement the receptor binding site (that is likely to be elastic depending on the ligand), fifty constructs will be tested for various attributes such as size (G-23, G-24), hydrophobicity, charge (G-17, G-18, G-23, polarizability (G-4, G-5, G-12, G-11, G-27)), stereochemistry (G-17, G-18, G-20, G-21) and electronegativity (G-4, G-8, G-9). While the predicted p*K*ₐ values of appendages that were tolerated in GLP-1 indicate that the positive charge is not essential, the native ligand is partially charged at physiological pH. Assuming that the "X" groups are accommodated in the binding pocket, the electron withdrawing and donating nature of the substituent will be varied at the 4-position on the aryl ring of the *N-*benzyl derivative [a total of 8 compounds ranging from the most electron withdrawing -NO₂ (Hammett σ = 0.81) to the most electron donating -N(CH₃)₂ (σ = -0.83)]. Plots of log(EC_{50,X}/EC_{50,H}) versus σ_{X} will be used to determine the slope *ρ* (a measure of the equilibrium's sensitivity to substituents relative to benzoic acid dissociation) as a thermodynamically assessed parameter. The p*K*ₐ of the *α-*amine is estimated to be 6.91 and can be altered in either direction depending on the identity of the X appendage.

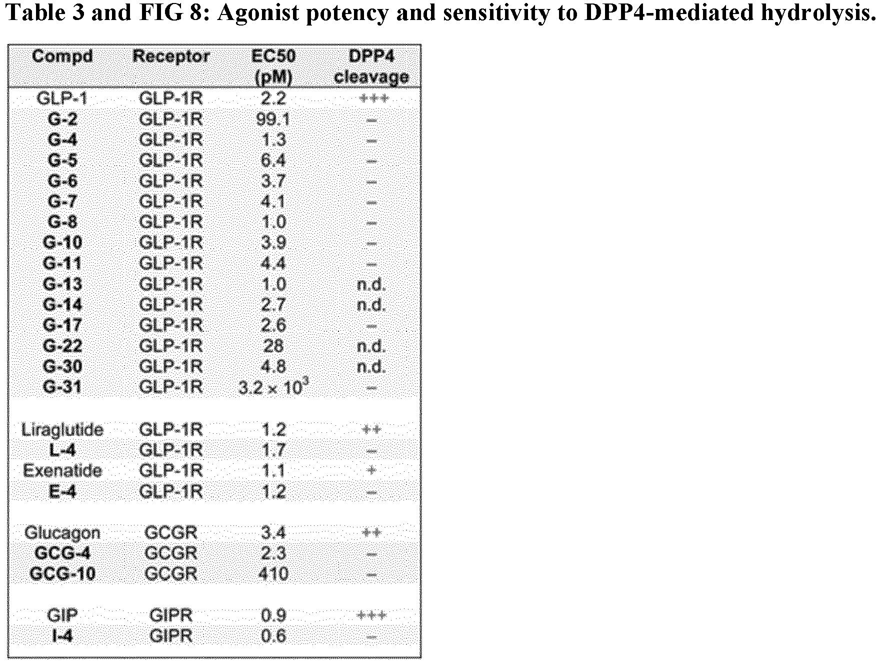

### Example 9: Impact of alkylation on the stability and function of glucagon.

The N-terminus of glucagon (His-Ser) is different than that of either GLP-1 (His-Ala) or GIP (Tyr-Ala). While the serine residue in the penultimate position of glucagon reduces DPP4 sensitivity of this peptide vs. either of the incretins, glucagon is still degraded by DPP4 and would benefit from N-terminal protection to extend its half life. The data has shown in experiments that attachment of a trifluoroethyl group affords complete DPP4 resistance of glucagon without compromising agonist potency (FIG. 13 and Table 3). The glucagon receptor has recently been crystallized (albeit at limited resolution and including only part of the molecule), however there remains uncertainty how the N-terminus of glucagon fits with its cognate GPCR. Following the experimental design outlined for GIP, which of 30 selected N-terminal decorations are tolerated in glucagon receptor will be determined, enabling an initial profile comparison of the agonist pockets of all three glucagon-family peptides to be studied.

### Example 17- GHRH Analogs (FIG. 6A)

FIG. 19 provides a series of graphs comparing sensitivity of native GHRH vs. CHCF3-GHRH ("C2-GHRH", bottom panel, compound GH29-4 in Fig. 6) enzymatic degradation. Activity of these peptides, tested as fresh stock or after overnight (O/N) incubation either with or without DPP4, was measured by luciferase assay in cells expressing GHRH receptors (GHRHR). In contrast to native GHRH, the GH29-4 derivative is resistant to enzyme induced potency loss (no rightward shift of concentration-response curve). GHRH analogs are useful to treat growth failure due to growth hormone deficiency (GHD), Growth failure in girls due to gonadal dysgenesis (Turner syndrome), Growth retardation in prepubertal children due to chronic renal disease, Growth disturbance (current height SDS < - 2.5 and parental adjusted height SDS < -1) in short children born small for gestational age (SGA), with a birth weight and/or length below -2 SD, who failed to show catch-up growth (HV SDS < 0 during the last year) by 4 years of age or later.

### Example 18: Analog Stability

Analogs of the invention are resistant to a variety of proteases. FIG. 18 provides a series of graphs comparing sensitivity of GLP-1 (left panel) vs. CHCF3-GLP-1 (right panel; G-4 in Fig. 6) to enzymatic degradation. Activity of these peptides, after overnight incubation without enzyme (control) or with either DPP4, DPP9, or FAP, was measured by luciferase assay in cells expressing GLP-1 receptors. In contrast to unmodified GLP-1, the G-4 derivative is resistant to enzyme induced potency loss (no rightward shift of concentration-response curve).

Not only are analogs of the invention resistant to proteases in vitro, but also demonstrate resistance in vivo relative to liraglutide. Fig. 22 provides a graph comparing the time-dependent decrease of plasma drug activity after a bolus injection of either liraglutide or a CHCF3-decorated derivative, Lira-4.
Rats received a bolus injection of either drug via central catheter, followed by serial blood draws after indicated intervals and measurement of plasma drug activity by bioassay of receptor agonism. Potency of each peptide immediately following injection was defined as 100%. Plasma survival of liraglutide is extended by the CHCF3 modification of Lira-4.

Fig. 22A demonstrates sustained hypoglycemic activity of Lira-4 following an oral glucose tolerance test. Mice received a s.c. injection of either vehicle, G-4, Liraglutide, or Lira-4 (represented from left to right in each group of bars). Half an hour and 5.5 hours later, two sequential oral glucose loads were applied by gavage. Blood glucose levels were measured just before drug injection (-30 minutes) and at indicated time intervals after the glucose loads. A single injection of compound G-4 attenuated glycemic excursion after the first glucose load, and still remained active to attenuate a second glucose challenge several hours later.

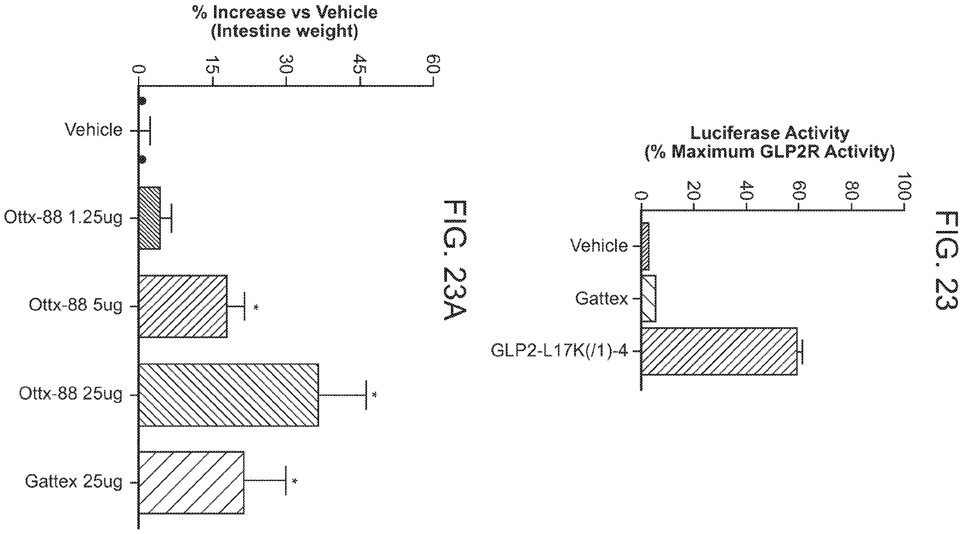

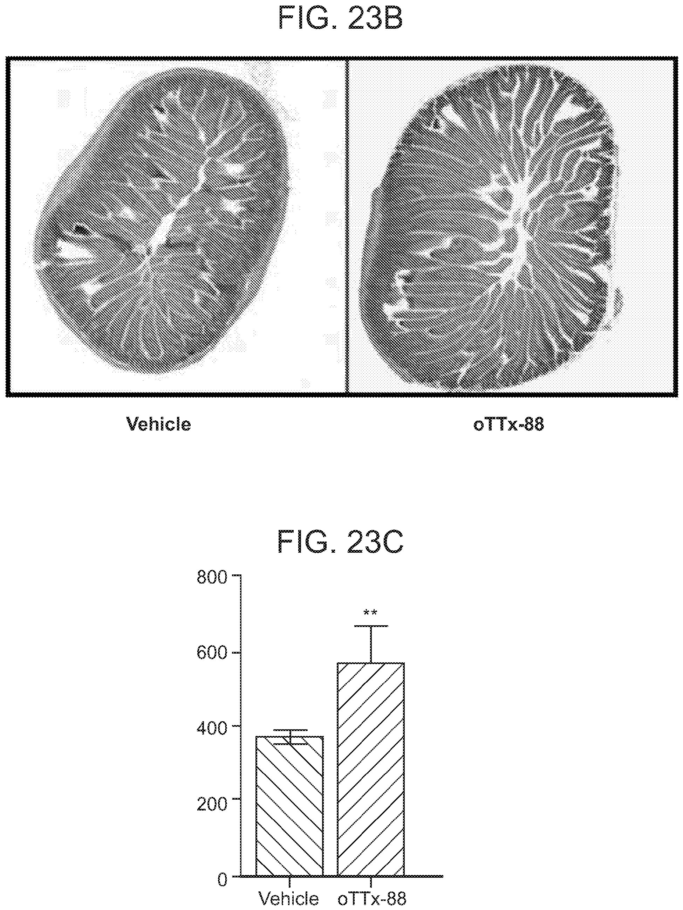

FIG. 23A provides a graph demonstrating extended bioactivity *in vivo* of GLP2-L17K(*l1*)-4, a fluorinated/acylated GLP-2 analogue shown in Fig. 6A. Clearance of this compound was compared to that of Gattex, an established GLP-2 based drug, compounds (1.25µg) were injected subcutaneously (s.c.) in mice. Plasma was collected after 24h and analyzed by luciferase reporter gene assay for GLP-2R agonist activity.

The results described herein above, were obtained using the following methods and materials.

Depending on the potency of the drug, there is a possibility that PET does not provide adequate resolution. If this situation arises, biodistribution is used instead of PET imaging. In biodistribution studies, following the administration of FDG (using a method similar to that described for PET-imaging), the mice are euthanized and intestinal segments are collected, weighed, and quickly transferred to a γ-counter to measure radioactivity. [¹⁸F]FDG tissue uptake levels are expressed as a percentage of injected dose per gram of tissue.

There is a distinction between promoting healthy epithelial regeneration, and accelerating growth of early stage adenomas or cancer. In the animal studies, histological sections are examined for abnormal growth.

The disclosures of each and every patent, patent application, and publication cited herein are hereby incorporated herein by reference in their entirety.

While the invention has been disclosed with reference to specific embodiments, it is apparent that other embodiments and variations of this invention may be devised by others skilled in the art without departing from the true spirit and scope of the invention. The appended claims are intended to be construed to include all such embodiments and equivalent variations.

## Claims

1. A chemically modified GLP-1 polypeptide, comprising an amino acid sequence having at least 75% identity to GLP-1 (H*AEGTFTS*DVS*S*YLEGQAAK*EFIAWLVK*GR), wherein at least one of the amino acid residues marked with * is alkylated with an optionally substituted C₁-C₁₆ alkyl group, wherein the N-terminal amino acid residue of the polypeptide is an N-2,2,2-trifluoroethyl amino acid represented by the formula:

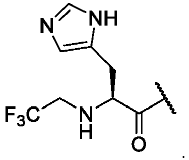

2. The chemically modified polypeptide of claim 1, wherein the polypeptide has 75% identity to GLP-1 (7-37) (His-Ala-Glu-Gly-Thr-Phe-Thr-Ser-Asp-Val-Ser-Ser-Tyr-Leu-Glu-Gly-Gln-Ala-Ala-Lys-Glu-Phe-Ile-Ala-Trp-Leu-Val-Lys-Gly-Arg-Gly-OH), wherein the lysine residue at position 26 (K26) has a lipid side chain (R = γ-Glu-palmitoyl); and Lys 34 (K34) is modified to an Arg residue (R34) having the structure:

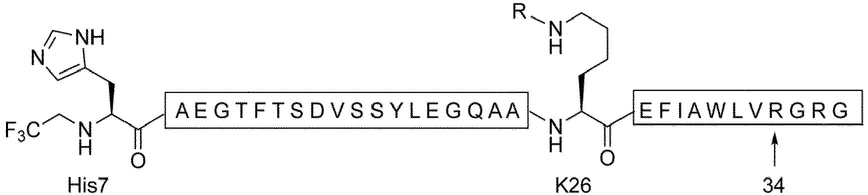

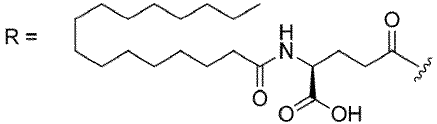

3. The polypeptide of claim 2, wherein the polypeptide is GLP-1 (7-37) (His-Ala-Glu-Gly-Thr-Phe-Thr-Ser-Asp-Val-Ser-Ser-Tyr-Leu-Glu-Gly-Gln-Ala-Ala-Lys-Glu-Phe-Ile-Ala-Trp-Leu-Val-Lys-Gly-Arg-Gly-OH), wherein the lysine residue at position 26 (K26) has a lipid side chain (R = γ-Glu-palmitoyl); and Lys 34 (K34) is modified to an Arg residue (R34), having the structure

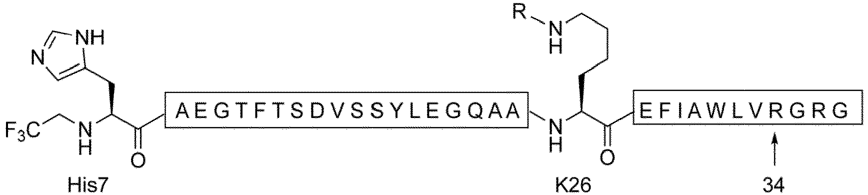

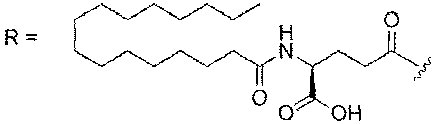

4. A pharmaceutically acceptable composition comprising at least one polypeptide of any of claims 1-3.

5. A polypeptide of any one of claims 1-3, or the pharmaceutically acceptable composition of claim 4, for use in a method of treating or preventing at least one disease or disorder selected from the group consisting of short bowel syndrome, Non-Alcoholic steatohepatitis, smoking cessation, neurodegeneration, Alzheimer's disease, Parkinson's disease, congenital hyperinsulism, hypoglycemia, diabetes, weight gain, obesity, and metabolic syndrome, comprising administering at least one polypeptide of any one of claims 1-3, or the pharmaceutically acceptable composition of claim 4.

6. A method of imaging a cell or tissue in a subject, the method comprising administering to the subject in need thereof an effective amount of a polypeptide of any one of claims 1-3 or the pharmaceutically acceptable composition of claim 4, wherein the polypeptide is labeled with a detectable isotope and/or conjugated to a detectable label, optionally, wherein the detectable label comprises a chromophore, a fluorescent group, a bioluminescent group, a chemiluminescent group or a radioactive group.

## Patentansprüche

1. Chemisch modifiziertes GLP-1-Polypeptid, das eine Aminosäuresequenz mit mindestens 75% Identität mit GLP-1 (H*AEGTFTS*DVS*S*YLEGQAAK*EFIAWLVK*GR) umfasst, wobei mindestens einer der mit * markierten Aminosäurereste mit einer optional substituierten C₁-C₁₆-Alkylgruppe alkyliert ist, wobei der N-terminale Aminosäurerest des Polypeptids eine N-2,2,2-trifluorethylaminosäure ist, dargestellt durch folgende Formel:

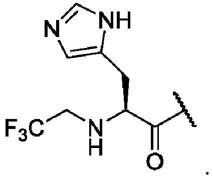

2. Chemisch modifiziertes Polypeptid nach Anspruch 1, wobei das Polypeptid 75% Identität mit GLP-1 (7-37) (His-Ala-Glu-Gly-Thr-Phe-Thr-Ser-Asp-Val-Ser-Ser-Tyr-Leu-Glu-Gly-Gln-Ala-Ala-Lys-Glu-Phe-Ile-Ala-Trp-Leu-Val-Lys-Gly-Arg-Gly-OH) aufweist, wobei der Lysinrest an Position 26 (K26) eine Lipidseitenkette (R = γ-Glu-palmitoyl) aufweist und Lys 34 (K34) zu einem Arg-Rest (R34) modifiziert ist, mit folgender Struktur:

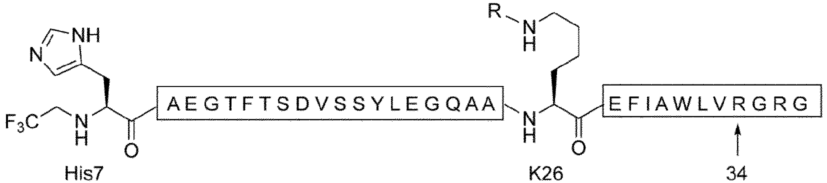

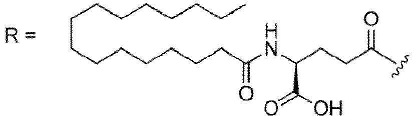

3. Polypeptid nach Anspruch 2, wobei das Polypeptid GLP-1 (7-37) (His-Ala-Glu-Gly-Thr-Phe-Thr-Ser-Asp-Val-Ser-Ser-Tyr-Leu-Glu-Gly-Gln-Ala-Ala-Lys-Glu-Phe-Ile-Ala-Trp-Leu-Val-Lys-Gly-Arg-Gly-OH) ist, wobei der Lysinrest an Position 26 (K26) eine Lipidseitenkette (R = γ-Glu-palmitoyl) aufweist und Lys 34 (K34) zu einem Arg-Rest (R34) modifiziert ist, mit folgender Struktur:

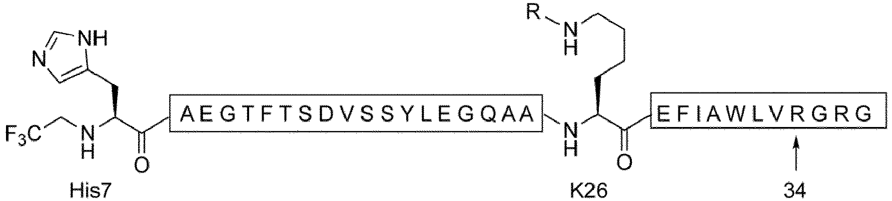

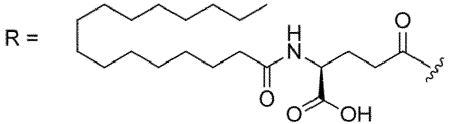

4. Pharmazeutisch akzeptable Zusammensetzung, die mindestens ein Polypeptid nach einem der Ansprüche 1 - 3 umfasst.

5. Polypeptid nach einem der Ansprüche 1 - 3, oder die pharmazeutisch akzeptable Zusammensetzung nach Anspruch 4, zur Verwendung in einem Verfahren zur Behandlung oder Prävention mindestens einer Krankheit oder Störung, die ausgewählt ist aus der Gruppe bestehend aus Kurzdarmsyndrom, Nicht-alkoholischer Steatohepatitis, Rauchentwöhnung, Neurodegeneration, Alzheimer-Krankheit, Parkinson-Krankheit, kongenitalem Hyperinsulinismus, Hypoglykämie, Diabetes, Gewichtszunahme, Adipositas und metabolischem Syndrom, umfassend die Verabreichung von mindestens einem Polypeptid nach einem der Ansprüche 1 - 3 oder der pharmazeutisch akzeptablen Zusammensetzung nach Anspruch 4.

6. Verfahren zur Abbildung einer Zelle oder eines Gewebes in einem Patienten, wobei das Verfahren umfasst, dem Patienten, der dessen bedarf, eine wirksame Menge eines Polypeptids nach einem der Ansprüche 1 - 3 oder der pharmazeutisch akzeptablen Zusammensetzung nach Anspruch 4 zu verabreichen, wobei das Polypeptid mit einem nachweisbaren Isotop markiert ist und/oder an eine nachweisbare Markierung konjugiert ist, wobei die nachweisbare Markierung optional einen Chromophor, eine fluoreszente Gruppe, eine biolumineszente Gruppe, eine chemilumineszente Gruppe oder eine radioaktive Gruppe umfasst.

## Revendications

1. Polypeptide GLP-1 modifié chimiquement, comprenant une séquence d'acide aminé possédant au moins 75 % d'identité par rapport au GLP-1 (H*AEGTFTS*DVS*S*YLEGQAAK*EFIAWLVK*GR), dans lequel au moins un des résidus d'acide aminé marqués d'une * est alkylé avec un groupe d'alkyle C₁-C₁₆ substitué en option, dans lequel le résidu d'acide aminé N-terminal du polypeptide est un acide aminé N-2,2,2-trifluoroéthylique représenté par la formule :

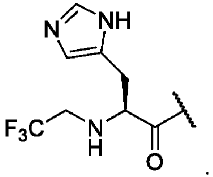

2. Polypeptide modifié chimiquement selon la revendication 1, dans lequel le polypeptide possède 75 % d'identité par rapport au GLP-1 (7-37) (His-Ala-Glu-Gly-Thr-Phe-Thr-Ser-Asp-ValSer-Ser-Tyr-Leu-Glu-Gly-Gln-Ala-Ala-Lys-Glu-Phe-Ile-Ala-Trp-Leu-Val-Lys-Gly-Arg-Gly-OH), dans lequel le résidu de lysine à la position 26 (K26) possède une chaîne côté lipide (R = γ-Glupalmitoyle) ; et la lys 34 (K34) est modifiée en un résidu Arg (R34) possédant la structure :

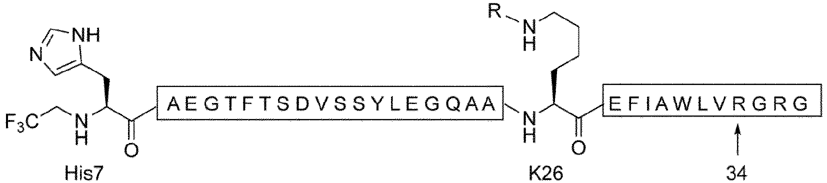

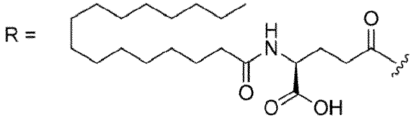

3. Polypeptide selon la revendication 2, dans lequel le polypeptide est du GLP-1 (7-37) (His-Ala-Glu-Gly-Thr-Phe-Thr-Ser-Asp-Val-Ser-Ser-Tyr-Leu-Glu-Gly-Gln-Ala-Ala-Lys-Glu-Phe-Ile-Ala-Trp-Leu-Val-Lys-Gly-Arg-Gly-OH), dans lequel le résidu de lysine à la position 26 (K26) possède une chaîne côté lipide (R = γ-Glu-palmitoyle) ; et la lys 34 (K34) est modifiée en un résidu Arg (R34) possédant la structure :

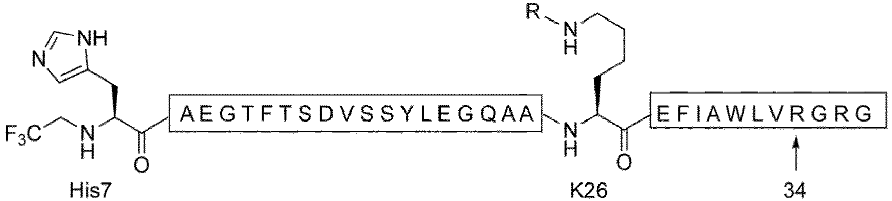

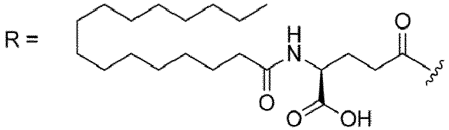

4. Composition pharmaceutiquement acceptable comprenant au moins un polypeptide selon une quelconque des revendications 1 à 3.

5. Polypeptide selon une quelconque des revendications 1 à 3, ou composition pharmaceutiquement acceptable selon la revendication 4, en vue de l'utilisation dans une méthode de traitement ou de prévention d'au moins une pathologie ou d'un trouble sélectionné parmi le groupe constitué par le syndrome de l'intestin court, la stéatohépatite non alcoolique, le sevrage tabagique, la neurodégénérescence, la maladie d'Alzheimer, la maladie de Parkinson, l'hyperinsulinisme congénital, l'hypoglycémie, le diabète, la prise de poids, l'obésité, et le syndrome métabolique, comprenant l'administration d'au moins un polypeptide selon une quelconque des revendications 1 à 3, ou de la composition pharmaceutiquement acceptable selon la revendication 4.

6. Méthode d'imagerie d'une cellule ou d'un tissu chez un sujet, la méthode comprenant l'administration au sujet qui en a besoin d'une quantité efficace d'un polypeptide selon une quelconque des revendications 1 à 3 ou de la composition pharmaceutiquement acceptable selon la revendication 4, dans laquelle le polypeptide est marqué avec un isotope détectable et/ou conjugué à un label détectable, en option dans laquelle le label détectable comprend un chromophore, un groupe fluorescent, un groupe bioluminescent, un groupe chimio-luminescent ou un groupe radioactif.
